# EUROPEAN PATENT SPECIFICATION

(11) **EP 1 115 403 B1**
(45) Date of publication and mention of the grant of the patent: **14.12.2005**
(21) Application number: 99969336.9
(22) Date of filing: 24.09.1999
(51) Int. Cl.: A61K 31/52, A61P 1/04

(54) **METHOD FOR OPTIMIZING THE USE OF 6-MERCAPTOPURINE IN THE TREATMENT OF IMMUNE-MEDIATED GASTROINTESTINAL DISORDERS**
METHODE ZUR OPTIMIERUNG DER VERWENDUNG VON 6-MERCAPTOPURIN IN DER BEHANDLUNG VON IMMUN-VERMITTELTEN GASTROINTESTINALEN STÖRUNGEN
METHODE POUR OPTIMISER L'UTILISATION D'UN MEDICAMENT CONTENANT DE LA 6-MERCAPTOPURINE DANS LE TRAITEMENT DE MALADIES GASTRO-INTESTINALES A MEDIATION IMMUNE

(30) Priority: 24.09.1998 US 101714 P; 08.04.1999 US 288344
(43) Date of publication of application: 18.07.2001
(73) Proprietor: Hopital Sainte-Justine, Montreal, QC H3T 1CS (CA)
(72) Inventor: SEIDMAN, Ernest G., Quebec, H4W 1T5 (CA); THEORET, Yves, Montreal, Quebec H3X 3N9 (CA)
(74) Representative: UEXKÜLL & STOLBERG
(86) International application number: PCT/IB1999/001691
(87) International publication number: WO 2000/016780

(56) References cited:
- WO-A-96/30021
- BARBARA S. KIRSCHNER: "Safety of Azathioprine and 6-Mercaptopurin in Pediatric Patients With Inflammatory Bowel Disease" GASTROENTEROLOGY, vol. 115, 1998, pages 813-821, XP002130481 usa cited in the application
- JAMES E.F. REYNOLDS: "MARTINDALE THE EXTRA PHARMACOPOEIA" 1993 , THE PHARMACUTICAL PRESS , LONDON, GB XP002130482 page 449, right-hand column

## Description

### BACKGROUND OF THE INVENTION

The present invention relates in vitro methods for optimizing treatment of immune-mediated gastrointestinal disorders.

### BACKGROUND INFORMATION

Immune-mediated gastrointestinal disorders encompass a wide range of debilitating gastrointestinal diseases of various etiologies. One such immune-mediated gastrointestinal disorder, inflammatory bowel disease (IBD), is the collective term used to describe two gastrointestinal disorders of unknown etiology: Crohn's disease (CD) and ulcerative colitis (UC). The course and prognosis of IBD, which occurs world-wide and is reported to afflict as many as two million people, varies widely. Onset of IBD is predominantly in young adulthood with diarrhea, abdominal pain, and fever the three most common presenting symptoms. The diarrhea may range from mild to severe and in ulcerative colitis often is accompanied by bleeding. Anemia and weight loss are additional common signs of IBD. Ten percent to fifteen percent of all patients with IBD will require surgery over a ten year period. In addition, patients with IBD are at increased risk for the development of intestinal cancer. Reports of an increasing occurrence of psychological problems, including anxiety and depression, are perhaps not surprising symptoms of what is often a debilitating disease that strikes people in the prime of life.

6-Mercaptopurine (6-MP) and azathioprine (AZA), a pro-drug that is non-enzymatically converted to 6-mercaptopurine (6-MP), are 6-MP drugs that can be used as an effective treatment for inflammatory bowel diseases such as Crohn's disease and ulcerative colitis (Kirschner Gastroenterology 115:813-821 (1998)). 6-MP can be enzymatically converted to various 6-MP metabolites, including 6-methyl-mercaptopurine (6-MMP) and 6-thioguanine (6-TG) and their nucleotides. 6-TG nucleotides are thought to be the active metabolite in mediating many of the effects of 6-MP drug treatment.

Thiopurine methyltransferase (TPMT) is a cytoplasmic enzyme that preferentially catalyzes the S-methylation of 6-MP and 6-TG to form S-methylated metabolites such as 6-MMP and 6-methylthioguanine (6-MTG), respectively. TPMT exhibits genetic polymorphism, with 89% of Caucasians and African Americans having high activity, 11% intermediate activity and 1 in 300 TPMT deficient. Clinical studies with AZA and 6-MP have shown an inverse relationship between TPMT activity and 6-TGN accumulation. Patients who less efficiently methylate these thiopurines have more extensive conversion to 6-TGN, which can lead to potentially fatal hematopoietic toxicity. Therefore, patients who have less active TPMT can be more susceptible to toxic side effects of 6-MP therapy.

Although drugs such as 6-MP and AZA have been used for treating IBD, non-responsiveness and drug toxicity unfortunately complicate treatment in some patients. Complications associated with 6-MP drug treatment include allergic reactions, neoplasia, opportunistic infections, hepatitis, bone marrow suppression, and pancreatitis. Therefore, many physicians are reluctant to treat patients with AZA because of its potential side effects, especially infection and neoplasia.

Thus, there exists a need to develop methods to optimize the dose of 6-mercaptopurine drugs and assess biotransformation in individual patients to optimize the therapeutic efficacy of 6-mercaptopurine drugs while minimizing toxic side effects. The present invention satisfies this need and provides related advantages as well.

### SUMMARY OF THE INVENTION

The present invention provides an in vitro method of optimizing therapeutic efficacy of treatment of an immune-mediated gastrointestinal disorder by administration of a 6-mercaptopurine drug. The method includes determining a level of 6-thioguanine in a sample form a subject having an immune-mediated gastrointestinal disorder, where a level of 6-thioguanine less than a level corresponding to about 230 pmol per 8x10⁸ red blood cells indicates a need to increase the amount of 6-mercaptopurine drug subsequently administered to the subject and where a level of 6-thioguanine greater than a level corresponding to about 400 pmol per 8x10⁸ red blood cells indicates a need to decrease the amount of 6-mercaptopurine drug subsequently administered to the subject. The methods are directed to treating immune-mediated gastrointestinal disorders, including inflammatory bowel diseases (IBD) such as Crohn's disease and ulcerative colitis, lymphocytic colitis, microscopic colitis, collagenous colitis, autoimmune enteropathy, allergic gastrointestinal disease and eosinophilic gastrointestinal disease. In a method of optimizing therapeutic efficacy of 6-mercaptopurine treatment of IBD, the subject having IBD can be, for example, a pediatric subject. The level of 6-thioguanine can be determined, for example, in red blood cells using high pressure liquid chromatography.

The present invention also provides an vitro method of reducing toxicity associated with treatment of an immune-mediated gastrointestinal disorder by administration of a 6-mercaptopurine drug. The method of reducing toxicity associated with 6-mercaptopurin drug treatment of an immune-mediated gastrointestinal disorder includes determining a level of a 6-mercaptopurine metabolite in a sample from a subject having the immune-mediated gastrointestinal disorder, where a level of the 6-mercaptopurine metabolite greater than a predetermined toxic level of the 6-mercaptopurine metabolite indicates a need to decrease the amount of 6-mercaptopurine drug subsequently administered to the subject, thereby reducing toxicity associated with 6-mercaptopurine drug treatment of the immune-mediated gastrointestinal disorder. In a method of the invention, the 6-mercaptopurine metabolite can be, for example, 6-thioguanine and the predetermined toxic level of 6-thioguanine can correspond, for example, to a level of about 400 pmol per 8x10⁸ red blood cells. Where the elevated 6-mercaptopurine metabolite is 6-thioguanine, the toxicity associated with 6-mercaptopurine treatment can be, for example, hematologic toxicity. The 6-mercaptopurine metabolite also can be a metabolite such as 6-methyl-mercaptopurine and the predetermined toxic level of 6-methyl-mercaptopurine can correspond, for example, to a level of about 7000 pmol per 8x10⁸ red blood cells. Where the elevated 6-mercaptopurine metabolite is 6-methyl-mercaptopurine, the toxicity associated with 6-mercaptopurine treatment can be, for example, hepatic toxicity.

Further provided by the invention is an in vitro method of optimizing therapeutic efficacy and reducing toxicity associated with treatment of an immune-mediated gastrointestinal disorder by administration of a 6-mercaptopurine drug. The method includes the steps determining a level of 6-thioguanine in a sample from a subject having an immune-mediated gastrointestinal disorder; and determining a level of 6-methyl-mercaptopurine in the subject having the immune-mediated gastrointestinal disorder, where a level of 6-thioguanine less than a predetermined minimal therapeutic level indicates a need to increase the amount of 6-mercaptopurine drug subsequently administered to the subject, thereby increasing therapeutic efficacy; where a level of 6-thioguanine greater than a predetermined toxic level of 6-thioguanine indicates a need to decrease the amount of 6-mercaptopurine drug subsequently administered to the subject, thereby reducing toxicity associated with 6-mercaptopurine treatment of the immune-mediated gastrointestinal disorder; and where a level of 6-methyl-mercaptopurine greater than a predetermined toxic level of 6-methyl-mercaptopurine indicates a need to decrease the amount of 6-mercaptopurine drug subsequently administered to the subject, thereby reducing toxicity associated with 6-mercaptopurine treatment of the immune-mediated gastrointestinal disorder.

In such a method of optimizing therapeutic efficacy and reducing toxicity associated with 6-mercaptopurine drug treatment of an immune-mediated gastrointestinal disorder, the predetermined minimal therapeutic level of 6-thioguanine can be, for example, a level corresponding to about 230 pmol per 8x10⁸ red blood cells; the predetermined toxic level of 6-thioguanine can be, for example, a level corresponding to about 400 pmol per 8x10⁸ red blood cells; and the predetermined toxic level of 6-methyl-mercaptopurine can be, for example, a level corresponding to about 7000 pmol per 8x10⁸ red blood cells. The level of 6-thioguanine and 6-methyl-mercaptopurine each can be conveniently determined, for example, in red blood cells using high pressure liquid chromatography. The invention further provides methods to optimize the therapeutic efficacy of 6-mercaptopurine drug treatment of a non-IBD autoimmune disease.

### BRIEF DESCRIPTION OF THE DRAWINGS

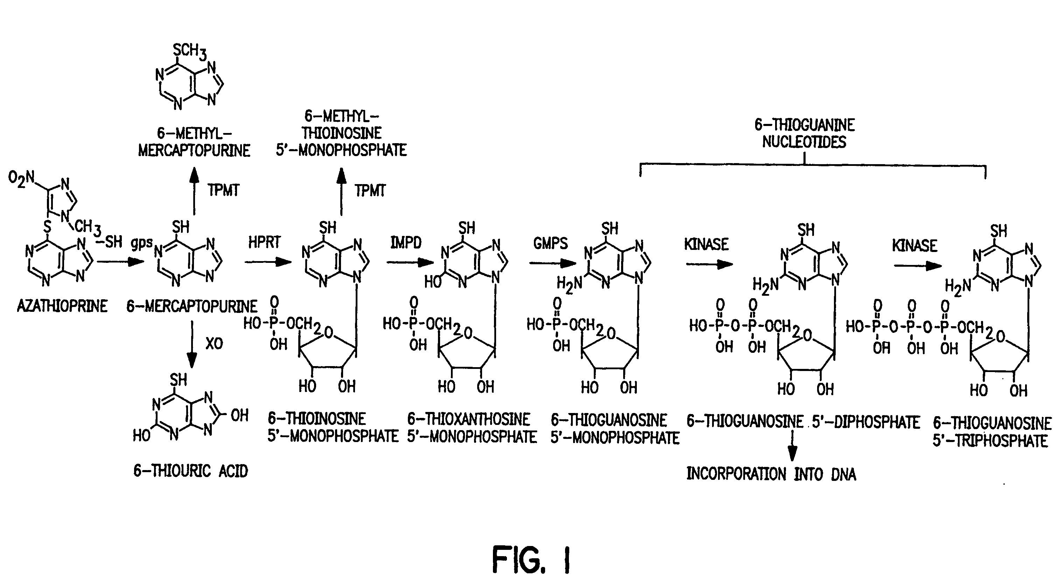

Figure 1 shows mercaptopurine metabolism and the structures of 6-mercaptopurine (6-MP) metabolites. The initial metabolism of 6-MP is catalyzed by thiopurine methyltransferase (TPMT), xanthine oxidase (XO), and hypoxanthine phosphoribosyltransferase (HPRT). Further metabolism of the thionucleotide is catalyzed by inosine monophosphate dehydrogenase (IMPD) and guanosine monophosphate synthetase (GMPS). The breakdown of azathioprine to 6-mercaptopurine is nonenzymatic.

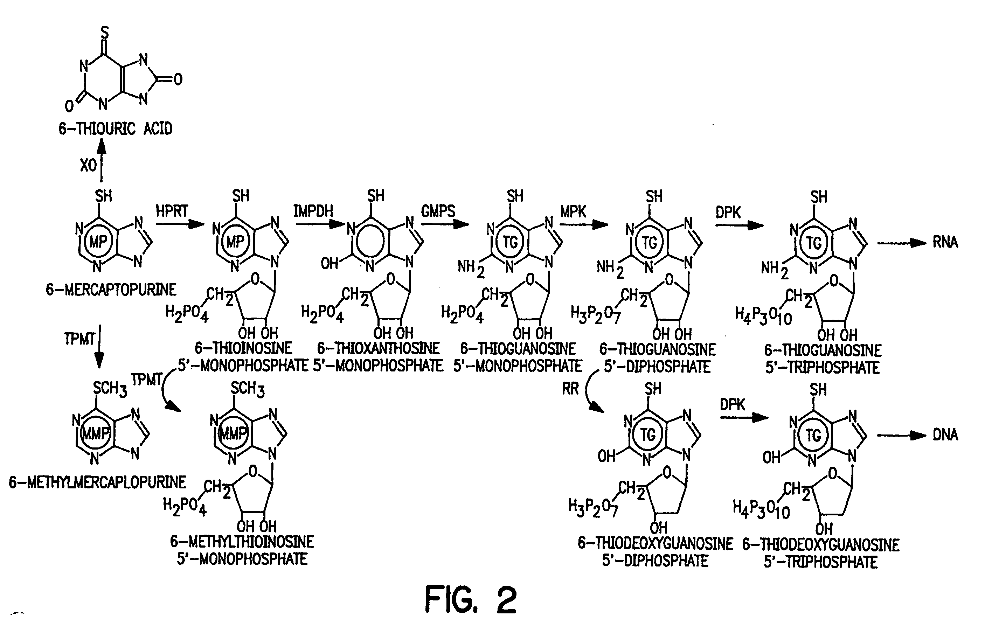

Figure 2 shows 6-mercaptopurine (6-MP) metabolism and the 6-MP metabolites that are measured as 6-MP, 6-thioguanine (6-TG) and 6-methyl-mercaptopurine (6-MMP)(indicated as "MP," "TG" and "MMP" inside the base). 6-TG mono-phosphate is converted to the di- and tri-phosphate by monophosphate kinase (MPK) and diphosphate kinase (DPK), respectively. The ribonucleoside diphosphate is converted to deoxyribonucleoside diphosphate by ribonucleotide reductase (RR).

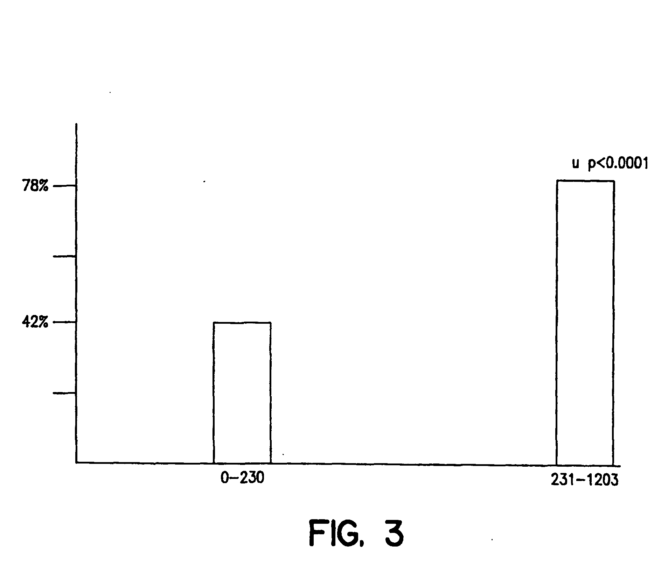

Figure 3 shows ranges of 6-thioguanine in IBD patients treated with a 6-MP drug.

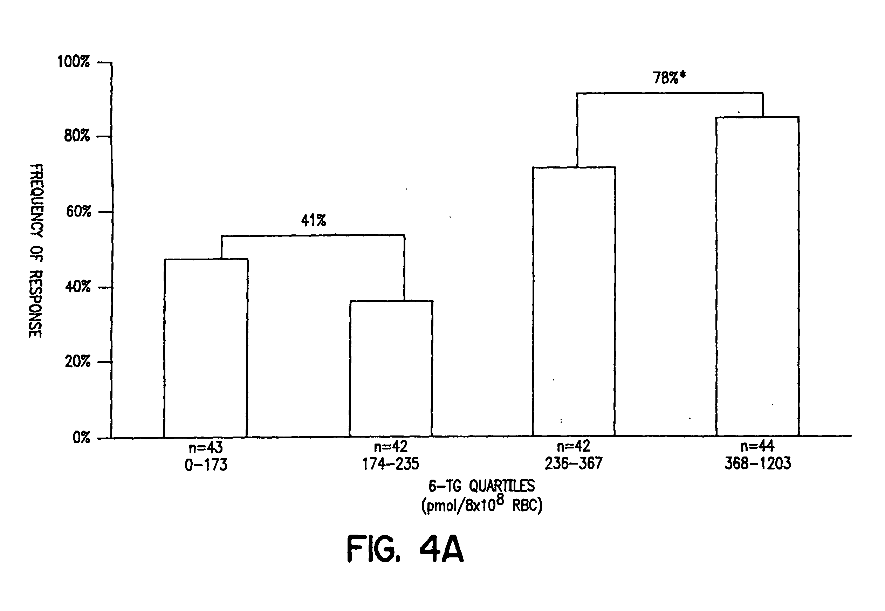

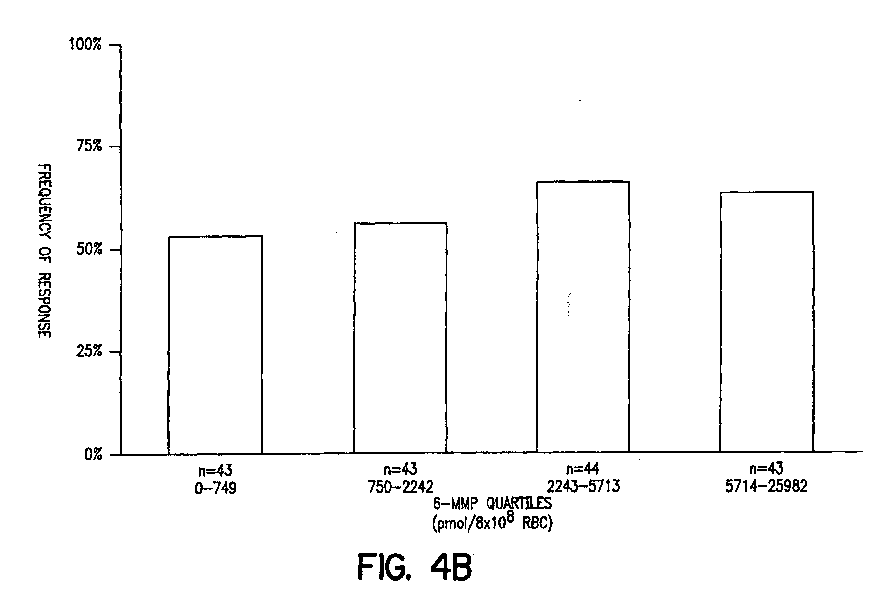

Figure 4 shows the correlation of quartile 6-MP metabolite levels with clinical response in IBD. Panel A shows the frequency of clinical response was significantly higher within the upper 6-TG level quartiles (>235 pmol/8x10⁸RBC)(*p<0.001). Panel B shows an association between 6-MMP quartile levels and clinical response. No significant differences were observed (p=0.48). n=number of clinical evaluation points per quartile.

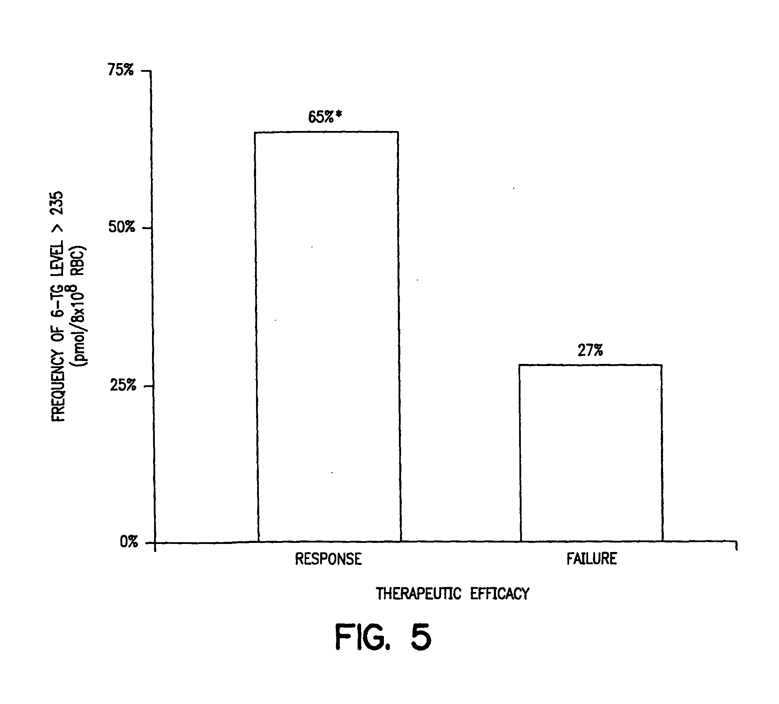

Figure 5 shows the frequency of 6-TG levels >235 pmol/8x10⁸ RBC at clinical evaluation points corresponding to therapeutic response, compared to points of therapeutic failure (*p<0.001).

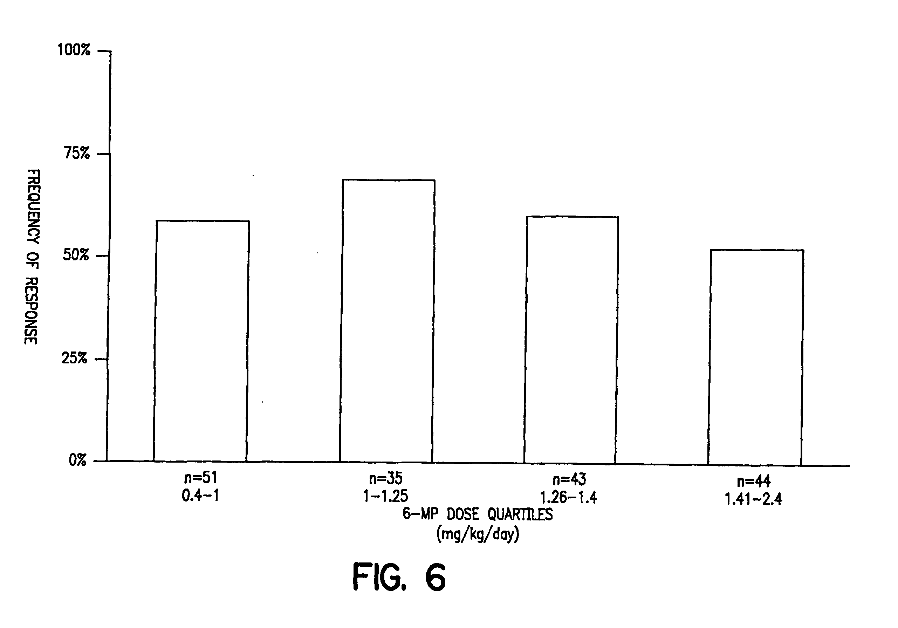

Figure 6 shows the relationship between 6-MP dose and clinical response in IBD. No significant differences were observed in the frequency of clinical response within 6-MP dose quartiles. AZA dose was converted to 6-MP dose by a factor of 2.07. n=number of clinical evaluation points per quartile.

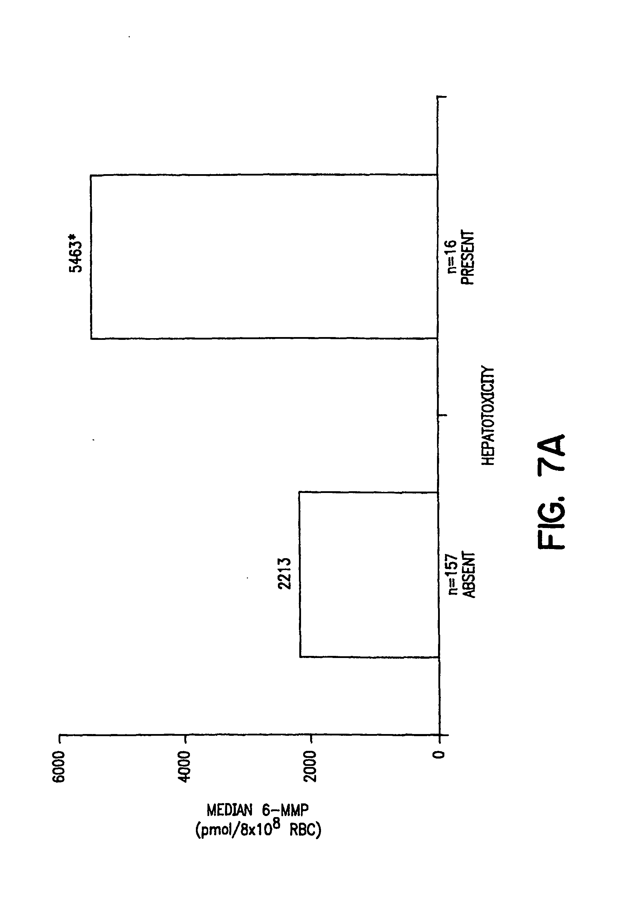

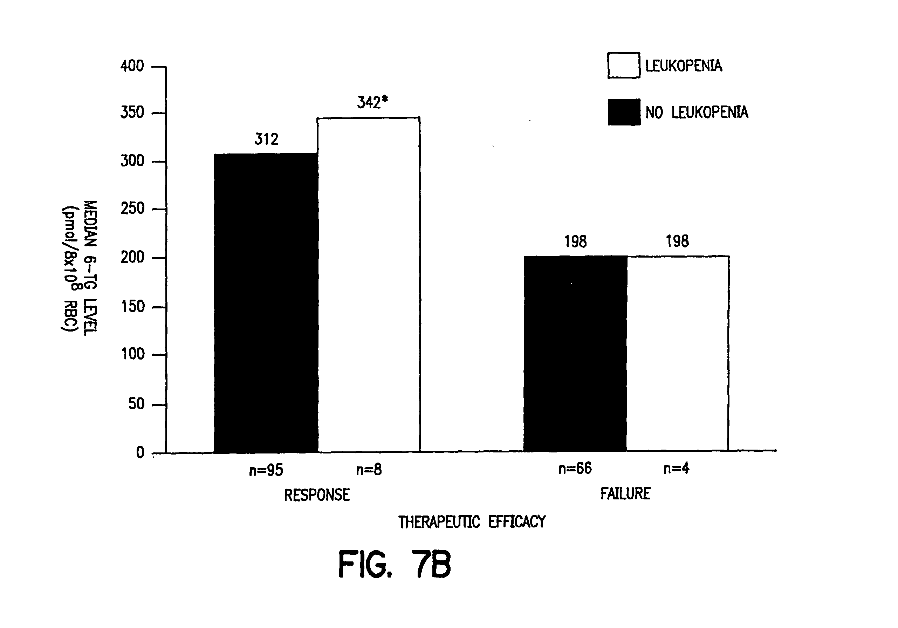

Figure 7 shows correlation of erythrocyte 6-MP metabolite levels with drug-related side effects in IBD. n=number of observations. Panel A shows the correlation of 6-MMP levels with hepatotoxicity. Hepatotoxic events were associated with higher median 6-MMP levels (*p<0.05). Panel B shows the correlation of 6-TG levels with leukopenia at times of therapeutic response and failure. The solid line for each group represents the median 6-TG level (*p=0.035, leukopenia vs. no leukopenia).

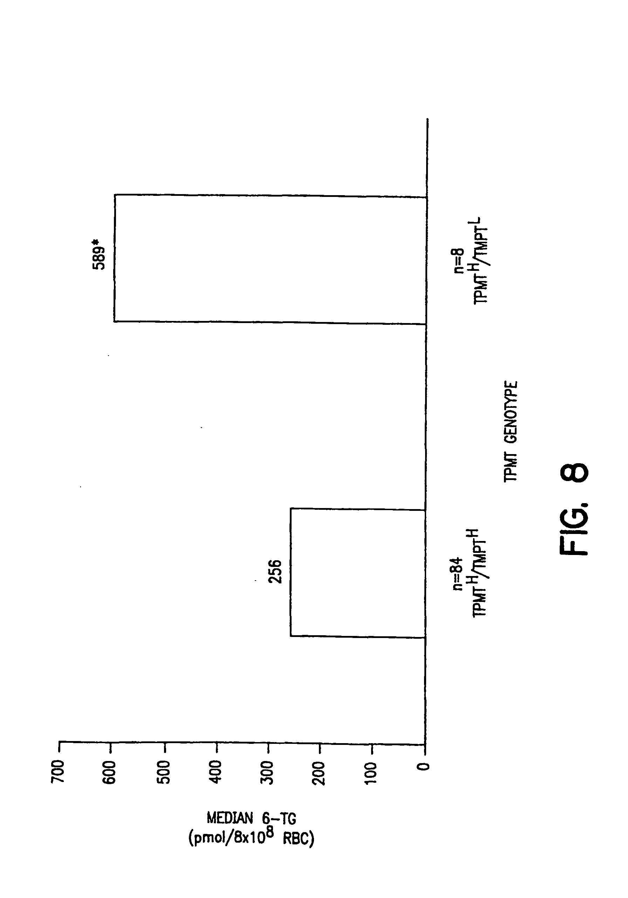

Figure 8 shows the relationship between TPMT genotype and 6-TG level in IBD. Median 6-TG levels were higher among heterozygotes (TPMT^{H}/ TMPT^{L}) (n=8) than homozygous normals (TPMT^{H}/TMPT^{H}) (n=85) with high activity (H) alleles. *p<0.0001

### DETAILED DESCRIPTION OF THE INVENTION

The present invention provides an in vitro method of optimizing therapeutic efficacy of treatment of an immune-mediated gastrointestinal disorder by administration of a 6-mercaptopurine (6-MP) drug. The method includes determining a level of 6-thioguanine (6-TG) in a sample from a subject having an immune-mediated gastrointestinal disorder, where a level of 6-TG less than a level corresponding to about 230 pmol per 8x10⁸ red blood cells indicates a need to increase the amount of 6-MP drug subsequently administered to the subject and where a level of 6-TG greater than a level corresponding to about 400 pmol per 8x10⁸ red blood cells indicates a need to decrease the amount of 6-MP drug subsequently administered to the subject. The methods are directed to treating immune-mediated gastrointestinal disorders, including inflammatory bowel diseases (IBD) such as Crohn's disease and ulcerative colitis, lymphocytic colitis, microscopic colitis, collagenous colitis, autoimmune enteropathy, allergic gastrointestinal disease and eosinophilic gastrointestinal disease. In a method of optimizing therapeutic efficacy of 6-MP treatment of IBD, the subject having IBD can be, for example, a pediatric subject. The level of 6-TG can be determined, for example, in red blood cells using high pressure liquid chromatography (HPLC).

The invention provides in vitro methods of optimizing therapeutic efficacy of 6-MP drug treatment of an immune-mediated gastrointestinal disorder. The methods of the invention are particularly useful for treating an immune-mediated gastrointestinal disorder such as IBD, including Crohn's disease and ulcerative colitis and subtypes thereof. The methods of the invention allow the clinician to provide an individually optimized dosage of a 6-MP drug so as to achieve a target level of a 6-MP metabolite in a particular patient having an immune-mediated gastrointestinal disorder, thereby optimizing the effectiveness of 6-MP drug therapy in the patient. The methods of the invention for optimizing therapeutic efficacy of 6-MP drug treatment involve determining the level of 6-TG in a sample from a patient having an immune-mediated gastrointestinal disorder. As disclosed herein, the level of 6-TG measured in a patient treated with a 6-MP drug was an indicator of the effectiveness of drug treatment. A level of at least 230 pmol 6-TG/Bx10⁸ red blood cells (RBC) was found in responders to drug therapy (see Examples I and II). These results indicate that determining the level of 6-TG can be used to assess whether a patient has a level of 6-TG that is sufficient to alleviate symptoms of an immune-mediated gastrointestinal disorder such as IBD, thus optimizing therapeutic efficacy.

As used herein, the term "6-mercaptopurine drug" or "6-MP drug" refers to any drug that can be metabolized to an active 6-mercaptopurine metabolite that has therapeutic efficacy such as 6-TG. Exemplary 6-mercaptopurine drugs as defined herein include 6-mercaptopurine (6-MP) and azathioprine (AZA). As illustrated in Figure 1, both of 6-MP and AZA can be metabolized to 6-mercaptopurine metabolites such as the exemplary 6-mercaptopurine metabolites shown in Figure 1, including 6-thioguanine (6-TG), 6-methyl-mercaptopurine (6-MMP) and 6-thiouric acid. (Lennard, Eur. J. Clin. Pharmacol. 43:329-339 (1992)).

Other 6-MP drugs include, for example, 6-methylmercaptopurine riboside and 6-TG (Loo et al., Clin. Pharmacol. Ther. 9:180-194 (1968); O'Dwyer et al., J. Natl. Cancer Inst. 83:1235-1240 (1991); Erb et al., Cancer Chemother. Pharmacol. 42:266-272 (1998); Lancaster et al., Br. J. Haematol. 102:439-443 (1998); Ingle et al., Am. J. Clin. Oncol. 20:69-72 (1997); Evans and Relling, Leuk. Res. 18:811-814 (1994)). 6-TG is a particularly useful 6-MP drug in patients having high TPMT activity. Patients exhibiting high TPMT activity are expected to more easily convert 6-MP drugs such as 6-MP and AZA to 6-MMP (see Figures 1 and 2). As disclosed herein, high levels of 6-MMP are associated with hepatotoxicity (see Examples I and II). Therefore, patients with high TPMT activity can be more susceptible to toxic effects of 6-MP drug therapy. By administering 6-TG, which is an active 6-MP metabolite associated with therapeutic efficacy (see Examples I and II), the toxicity that can be associated with conversion of 6-MP to 6-MMP is bypassed.

It is understood that the 6-MP metabolites can be the metabolites shown in Figure 1 or analogues thereof. As used herein, the term "6-thioguanine" or "6-TG" refers to 6-thioguanine or analogues thereof, including molecules having the same base structure, for example, 6-thioguanine ribonucleoside, 6-thioguanine ribonucleotide mono-, di- and tri-phosphate, 6-thioguanine deoxyribonucleoside and 6-thioguanine deoxyribonucleotide mono-, di, and triphosphate. The term "6-TG" also includes derivatives of 6-thioguanine, including chemical modifications of 6-TG, so long as the structure of the 6-TG base is preserved.

As used herein, the term "6-methyl-mercaptopurine" or "6-MMP" refers to 6-methyl-mercaptopurine or analogues thereof, including analogues having the same base structure, for example, 6-methyl-mercaptopurine ribonucleoside, 6-methyl-mercaptopurine ribonucleotide mono-, di-, and tri-phosphate, 6-methyl-mercaptopurine deoxyribonucleoside, and 6-methyl-mercaptopurine deoxyribonucleotide mono-, di- and tri-phosphate. The term "6-MMP" also includes derivatives of 6-methyl-mercaptopurine, including chemical modifications of 6-MMP, so long as the structure of the 6-MMP base is preserved.

The methods of the invention relate to the optimization of treatment of an immune-mediated gastrointestinal disorder. As used herein, the term "immune-mediated gastrointestinal disorder" or "immune-mediated GI disorder" refers to a non-infectious disease of the gastrointestinal tract or bowel that is mediated by the immune system or cells of the immune system. Immune-mediated gastrointestinal disorders include, for example, inflammatory bowel diseases (IBD) such as Crohn's disease and ulcerative colitis, lymphocytic colitis, microscopic colitis, collagenous colitis, autoimmune enteropathy, allergic gastrointestinal disease and eosinophilic gastrointestinal disease.

The methods of the invention are particularly useful for optimizing the treatment of IBD, or subtypes thereof, which has been classified into the broad categories of Crohn's disease and ulcerative colitis. As used herein, "a subject having inflammatory bowel disease" is synonymous with the term "a subject diagnosed with having an inflammatory bowel disease," and means a patient having Crohn's disease or ulcerative colitis. Crohn's disease (regional enteritis) is a disease of chronic inflammation that can involve any part of the gastrointestinal tract. Commonly, the distal portion of the small intestine (ileum) and cecum are affected. In other cases, the disease is confined to the small intestine, colon or anorectal region. Crohn's disease occasionally involves the duodenum and stomach, and more rarely the esophagus and oral cavity.

The variable clinical manifestations of Crohn's disease are, in part, a result of the varying anatomic localization of the disease. The most frequent symptoms of CD are abdominal pain, diarrhea and recurrent fever. CD is commonly associated with intestinal obstruction or fistula, which is an abnormal passage between diseased loops of bowel, for example. Crohn's disease also includes complications such as inflammation of the eye, joints and skin; liver disease; kidney stones or amyloidosis. In addition, CD is associated with an increased risk of intestinal cancer.

Several features are characteristic of the pathology of Crohn's disease. The inflammation associated with CD, known as transmural inflammation, involves all layers of the bowel wall. Thickening and edema, for example, typically also appear throughout the bowel wall, with fibrosis also present in long-standing disease. The inflammation characteristic of CD also is discontinuous in that segments of inflamed tissue, known as "skip lesions," are separated by apparently normal intestine. Furthermore, linear ulcerations, edema, and inflammation of the intervening tissue lead to a "cobblestone" appearance of the intestinal mucosa, which is distinctive of CD.

A hallmark of Crohn's disease is the presence of discrete aggregations of inflammatory cells, known as granulomas, which are generally found in the submucosa. Some Crohn's disease cases display the typical discrete granulomas, while others show nonspecific transmural inflammation. As a result, the presence of discrete granulomas is indicative of CD, although the absence of granulomas also is consistent with the disease. Thus, transmural or discontinuous inflammation, rather than the presence of granulomas, is a preferred diagnostic indicator of Crohn's disease (Rubin and Farber, Pathology (Second Edition) Philadelphia: J.B. Lippincott Company (1994)).

Ulcerative colitis (UC) is a disease of the large intestine characterized by chronic diarrhea with cramping abdominal pain, rectal bleeding, and loose discharges of blood, pus and mucus. The manifestations of ulcerative colitis vary widely. A pattern of exacerbations and remissions typifies the clinical course of most UC patients (70%), although continuous symptoms without remission are present in some patients with UC. Local and systemic complications of UC include arthritis, eye inflammation such as uveitis, skin ulcers and liver disease. In addition, ulcerative colitis and especially long-standing, extensive disease is associated with an increased risk of colon carcinoma.

Several pathologic features characterize UC in distinction to other inflammatory bowel diseases. Ulcerative colitis is a diffuse disease that usually extends from the most distal part of the rectum for a variable distance proximally. The term left-sided colitis describes an inflammation that involves the distal portion of the colon, extending as far as the splenic flexure. Sparing of the rectum or involvement of the right side (proximal portion) of the colon alone is unusual in ulcerative colitis. The inflammatory process of ulcerative colitis is limited to the colon and does not involve, for example, the small intestine, stomach or esophagus. In addition, ulcerative colitis is distinguished by a superficial inflammation of the mucosa that generally spares the deeper layers of the bowel wall. Crypt abscesses, in which degenerated intestinal crypts are filled with neutrophils, also are typical of ulcerative colitis (Rubin and Farber, *supra*, 1994).

In comparison with Crohn's disease, which is a patchy disease with frequent sparing of the rectum, ulcerative colitis is characterized by a continuous inflammation of the colon that usually is more severe distally than proximally. The inflammation in ulcerative colitis is superficial in that it is usually limited to the mucosal layer and is characterized by an acute inflammatory infiltrate with neutrophils and crypt abscesses. In contrast, Crohn's disease affects the entire thickness of the bowel wall with granulomas often, although not always, present. Disease that terminates at the ileocecal valve, or in the colon distal to it, is indicative of ulcerative colitis, while involvement of the terminal ileum, a cobblestone-like appearance, discrete ulcers or fistulas suggest Crohn's disease.

In addition to IBD, immune-mediated GI disorders also include other gastrointestinal diseases such as lymphocytic colitis; microscopic colitis; collagenous colitis; autoimmune enteropathy, including autoimmune enteritis and autoimmune enterocolitis; allergic gastrointestinal disease; and eosinophilic gastrointestinal disease, including eosinophilic gastroenteritis and eosinophilic enteropathy.

Over the past two decades, the histological evaluation of colorectal biopsies obtained by colonoscopy has expanded the spectrum of chronic IBD. A new group of immune-mediated bowel disorders has emerged, characterized by chronic watery diarrhea, minimal or absent endoscopic findings, and inflammatory changes in mucosal biopsies. Lymphocytic colitis, also commonly referred to as microscopic colitis, is a clinicopathological syndrome characterized primarily by lymphocytic infiltration of the epithelium. Collagenous colitis is defined by the presence of a collagenous band below the surface epithelium, accompanied by an increase in inflammatory cell infiltrate (Lazenby et al. Hum. Pathol. 20:18-28 (1989)). These disorders are often associated with other autoimmune diseases such as rheumatoid arthritis, pernicious anemia, thyroiditis, uveitis and type I diabetes mellitus. Clinicians have used immunosuppressive drugs, including 6-MP, to treat these disorders (Deslandres et al. J. Pediatr. Gastroenterol. Nutr. 25:341-346 (1997)).

Autoimmune enteropathy, including autoimmune enteritis and autoimmune enterocolitis, is a syndrome of severe secretory diarrhea and marked enterocolitis, in association with diagnostic circulating antibodies to enterocytes (Seidman et al., J. Pediatr. 117:929-932 (1990)). This syndrome, most often seen in infancy, can be seen in association with other autoimmune diseases. Complete villous atrophy is associated with a severe inflammatory reaction on small bowel biopsies. Although some cases remit after an extended period of time, most patients die without immunosuppressive therapy, which can include 6-MP drug therapy.

Eosinophilic gastrointestinal disease, including eosinophilic gastroenteritis and eosinophilic enteropathy, is characterized by a dense infiltration of eosinophils in one or more areas of the gastrointestinal tract, variable intestinal symptoms, and usually a peripheral eosinophilia (80% of cases). Food allergic, including allergic gastrointestinal disease, and eosinophilic disorders of the gastrointestinal tract are commonly treated by dietary elimination of the offending nutrients. However, both food induced and eosinophilic enteropathies may, in certain circumstances, require corticosteroid and immunosuppressive therapy, including 6-MP (Russo et al., Pediatric Dev. Path. 2:65-71 (1999)).

The methods of the invention relate to optimizing therapeutic efficacy of 6-MP drug treatment of an immune-mediated GI disorder, including IBD such as Crohn's disease and ulcerative colitis and subtypes thereof. The methods of the invention are particularly useful for optimizing the treatment of patients dependent on steroid therapy for maintenance of remission of disease in Crohn's disease and ulcerative colitis patients. As used herein, the phrase "optimizing therapeutic efficacy of 6-MP drug treatment" refers to adjusting the therapeutic dosage of a 6-MP drug such as 6-MP or azathioprine so that the concentration of a 6-MP metabolite that is correlated with effective treatment is maintained. As set forth above, the methods of the invention allow the clinician to provide an individually optimized dosage of a 6-MP drug so as to achieve a target level of a 6-MP metabolite in a particular patient, thereby optimizing the effectiveness of 6-MP drug therapy in the patient. Therapeutic efficacy generally is indicated by alleviation of one or more signs or symptoms associated with the disease. In the case of immune-mediated GI disorders, in particular IBD, therapeutic efficacy is indicated by alleviation of one or more signs or symptoms associated with the disease, including, for example, joint pain, arthritis, arthalgia, anorexia, growth failure, fistula closure, abdominal pain, diarrhea, recurrent fever, anemia, weight loss, rectal bleeding, inflammation of the intestine, and loose discharges of blood, pus and mucus. Methods for determining therapeutic efficacy, in particular for treating IBD, are disclosed herein in Examples I and II.

Therapeutic efficacy can be readily determined by one skilled in the art as the alleviation of one or more signs or symptoms of the disease being treated. In the case of IBD, patients can be analyzed using a Crohn's disease activity index (Best et al., Gastroenterology 70:439-444 (1976)). IBD patients can also be analyzed using a Harvey-Bradshaw index (HBI)(Harvey and Bradshaw, Lancet 1:514 (1980)). The Harvey-Bradshaw index provides an analytical method for measuring signs or symptoms of Crohn's disease, including the signs or symptoms of general well-being, abdominal pain, number of liquid stools per day, abdominal mass, and complications such as arthralgia, uveitis, erythema nodosum, aphthous ulcers, pyoderma gangrenosum, anal fissure, new fistula and abscess. The Harvey-Bradshaw index is particularly useful when evaluating pediatric patients.

Previous studies suggested that measurement of 6-MP metabolite levels can be used to predict clinical efficacy and tolerance to azathioprine or 6-MP (Cuffari et al., Gut 39:401-406 (1996a)). However, it was unknown what concentrations of 6-MP metabolites correlated with optimized therapeutic efficacy or with toxicity (Cuffari et al., *supra,* 1996a). As disclosed herein, levels of 6-MP metabolites such as 6-TG and 6-MMP were determined and correlated with therapeutic efficacy and toxicity associated with 6-MP drug therapy (see Examples I and II).

The invention is directed to in vitro methods of optimizing therapeutic efficacy of 6-MP drug treatment of an immune-mediated GI disorder by monitoring predetermined levels associated with therapeutic efficacy or toxicity and adjusting the 6-MP drug dosage so as to maintain an optimized dose that is efficacious and has reduced toxicity. The methods involve determining a level of a 6-MP metabolite in a sample from a subject having an immune-mediated GI disorder. The methods of the invention are advantageous in that the dosage of a 6-MP drug can be adjusted to maximize the efficacy of treating an immune-mediated GI disorder such as IBD while minimizing toxicity associated with 6-MP drug treatment.

As used herein, the term "6-mercaptopurine metabolite" refers to a product derived from 6-mercaptopurine in a biological system. Exemplary 6-mercaptopurine metabolites are shown in Figure 1 and include 6-thioguanine (6-TG), 6-methyl-mercaptopurine (6-MMP) and 6-thiouric acid and analogues thereof. For example, 6-MP metabolites include 6-TG bases such as 6-TG, 6-thioguanosine mono-, di- and tri-phosphate; 6-MMP bases such as 6-methyl-mercaptopurine and 6-methyl-thioinosine monophosphate; 6-thioxanthosine (6-TX) bases such as 6-thioxanthosine mono-phosphate; 6-thioruric acid (6-TUA); and 6-MP bases such as 6-mercaptopurine and 6-thioinosine monophosphate (see Figure 2). The immunosuppressive properties of 6-MP are believed to be mediated via the intracellular transformation of 6-MP to its active metabolites such as 6-TG and 6-MMP nucleotides. Furthermore, 6-MP metabolites such as 6-TG and 6-MMP were found to correlate with therapeutic efficacy and toxicity associated with 6-MP drug treatment of IBD patients (see Examples I and II).

The level of a 6-MP metabolite can be determined by methods well known in the art including, for example, those described in Lilleyman and Lennard, Lancet 343:1188-1190 (1994); Lennard and Singleton, J. Chromatography Biomed. Applicat. 583:83-90 (1992); Lennard and Singleton, J. Chromatography 661:25-33 (1994); and Cuffari et al., Can. J. Physiol. Pharmacol. 74:580-585 (1996b)). 6-MP metabolites such as 6-TG and 6-MMP can be measured, for example, by collecting red blood cells and extracting thiobases, for example, 6-MP, 6-TG, 6-TX and 6-MMP, which are released by acid hydrolysis. 6-MMP is converted to a form extractable by phenyl mercury salts (Dervieux and Boulieu, Clin. Chem. 44:2511-2525 (1998); Duchesne et al., Proc. Amer. Soc. Clin. Oncol. 13:137 (1994a); Duchesne et al., Can. J. Physiol. Pharmacol. 72:197 (1994b)). Such an analysis measures the thiobase and its analogues, including ribonucloside, ribonucleotide, deoxyribonucleoside, deoxyribonucleotide thiobases as well as mono-, di- and tri-phosphate analogues, which have been converted to thiobases.

Acid hydrolyzed extracts can be analyzed by resolving 6-MP metabolites and measuring their levels. For example, HPLC such as reverse phase HPLC is a useful method for resolving and measuring the levels of 6-MP metabolites, including 6-MP, 6-TG and 6-MMP (Lennard and Singleton, *supra,* 1992). Ultraviolet light (UV) detection can be used to measure the 6-MP metabolites. A particularly useful method of measuring 6-MP metabolites is isocratic reverse phase HPLC with UV detection (Cuffari et al., *supra*, 1996b).

Other methods for measuring 6-MP metabolites can also be used. For example, ion-pairing HPLC with dual UV-wavelength detection can be used to measure 6-MP metabolites (Zimm and Strong, Anal. Biochem. 160:1-6 (1987)). Additional methods for measuring 6-MP metabolites include, for example, capillary electrophoresis with laser-induced fluorescence detection (Rabel et al., Anal. Biochem. 224:315-322 (1995)); anion exchange chromatography and fluorescent detection (Tidd and Dedhar J. Chromatography 145:237-246 (1978)); lanthanum precipitation, acid hydrolysis, back extraction and fluorometric assay (Fletcher and Maddocks, Brit. J. Clin. Pharmacol. 10:287-292 (1980)); thin layer chromatography (Bennet and Allen, Cancer Res. 31:152-158 (1971)); precolumn derivatization with the thiol-reactive fluorophore monobromobimane, treatment with alkaline phosphatase, HPLC resolution and quantification by fluorometry (Warren and Slordal, Anal. Biochem. 215:278-283 (1993)); and enzymatic hydrolysis followed by HPLC separation and UV detection (Giverhaug et al., Ther. Drug Monit. 19:663-668 (1997)). 6-MP metabolites such as 6-TG can also be measured in DNA by degrading DNA to deoxyribonucleosides, derivatizing deoxy-6-TG with a fluorophore and resolving on reverse phase HPLC (Warren et al., Cancer Res. 55:1670-1674 (1995)).

As used herein, the level of a 6-MP metabolite can include the 6-MP metabolite itself, or the level of the 6-MP metabolite and analogues thereof. For example, as described above, acid hydrolysis can be used to release thionucleotides from a sample, resulting in conversion of mono-, di- and tri-phosphates to thiobases. In such an analysis, the level of several analogues of a thionucleotide are measured (see Figure 2). For example, measuring 6-TG can include 6-thioguanosine 5'-mono-, di-, and tri-phosphate, and 6-thiodeoxyguanosine 5'-di-, and tri-phosphate. Measuring 6-MP can include 6-mercaptopurine and 6-thioinosine 5'-monophosphate. Measuring 6-MMP can include 6-methylmercaptopurine and 6-methylthioinosine 5'-monophosphate, and can also include 6-methylthioinosine di- and tri-phosphate, as well as 6-methyl thioguanosine.

A particularly useful determination of the level of a 6-MP metabolite is the median level of the 6-MP metabolite since the distribution of 6-MP metabolite values is trimodal. Unless otherwise designated, the levels referred to herein are median levels. A 6-MP metabolite level can also be a mean level, if so desired. Unless otherwise designated, the levels of 6-MP metabolites referred to herein are values per 8x10⁸ RBC, whether reported as mean or median values.

6-MP metabolite levels can be conveniently assayed using red blood cells because such cells are readily available from the patient, lack a nucleus and are easy to manipulate. However, it should be understood that any measurement that allows determination of 6-MP metabolite levels can be used. For example, leukocytes can be used to measure 6-MP metabolite levels, which can be correlated with 6-MP metabolite levels in erythrocytes (Cuffari et al., *supra,* 1996b). Regardless of the method employed to measure 6-MP metabolites, one skilled in the art can readily measure 6-MP metabolite levels in a sample, for example, in leukocytes or DNA obtained from a patient, and correlate the level of 6-MP metabolites to the values disclosed herein, which were determined using RBC.

For convenience, levels of 6-MP metabolites disclosed herein are given in terms of an assay with RBC and, therefore, are given as an amount of a 6-MP metabolite in a given number of RBCs. 6-MP metabolites assayed in RBCs can also be determined relative to the amount of hemoglobin. However, it should be understood that one skilled in the art can measure 6-MP metabolite levels in samples other than RBCs and readily correlate such levels with 6-MP metabolite levels in RBCs. For example, one skilled in the art can readily determine levels of a 6-MP metabolite in cells such as leukocytes, or cells from the oral mucosa, and in RBCs by measuring the level of 6-MP metabolites in both types of cells and determining the correspondence between levels of 6-MP metabolites in RBCs and the levels in the other cells. Once a correspondence between 6-MP metabolites in RBCs and in a sample has been determined, one skilled in the art can use that correspondence to measure levels in the other sample and correlate those levels with the levels in RBCs disclosed herein.

As disclosed herein, the level of 6-TG in an IBD patient treated with a 6-MP drug was found to correlate with therapeutic efficacy (see Examples I and II). In particular, a median level of 230 pmol 6-TG/8x10⁸ RBC or more was found in IBD patients who responded to 6-MP drug therapy. Thus, as disclosed herein, a level of at least about 230 pmol 6-TG per 8x10⁸ RBC can be a minimal therapeutic level of 6-TG for efficaciously treating a patient (Examples I and II). For example, a minimal therapeutic level of 6-TG can be about 235 pmol 6-TG per 8x10⁸ RBC (see Example II). Accordingly, a level of 6-TG below about 230 pmol/8x10⁸ RBC indicates a need to increase the amount of a 6-MP drug administered to the patient. A minimal therapeutic level of 6-TG for efficaciously treating a patient also can be, for example, at least about 240 pmol per 8x10⁸ RBC; at least about 250 pmol per 8x10⁸ RBC; at least about 260 pmol per 8x10⁸ RBC; at least about 280 mol per 8x10⁸ RBC or at least about 300 pmol per 8x10⁸ RBC. It is understood that the minimal therapeutic levels of 6-TG disclosed herein are useful for treating immune-mediated gastrointestinal disorders, including IBD, as well as non-IBD autoimmune diseases.

As disclosed herein, erythrocyte 6-TG concentration, not drug dose, is significantly and independently associated with therapeutic response (see Example II). The results indicate that response to 6-MP drug therapy is optimized at 6-TG levels greater than about 230 pmol per 8x10⁸ RBC, for example, greater than about 230 pmol per 8x10⁸ RBC (see Example II).

As further disclosed herein, 6-MP metabolites are associated with toxicity (Example II). For example, hepatotoxicity correlated with the presence of significantly higher concentrations of 6-MMP (Example II). Monitoring 6-MMP levels is useful in identifying patients at risk for hepatotoxicity. Monitoring 6-MMP levels can also be used to determine the level at which a hepatic event has occurred in order to individualize appropriate dose adjustments to optimize the therapeutic dose. Moreover, 6-MMP monitoring can be performed in conjunction with liver function tests.

Furthermore, as disclosed herein, leukopenia was associated with higher 6-TG levels (see Example II). The median 6-TG level was 342 pmol per 8x10⁸ RBC (mean 490) in leukopenic individuals, well above the threshold level of 235 pmol per 8x10⁸ RBC. However, leukopenia is not a reliable marker for therapeutic response and is not required to control IBD (Example II).

As exemplified herein, 6-MP metabolite measurements can provide clinicians with useful methods to optimize 6-MP drug therapy for individuals having immune-mediated gastrointestinal disorders and IBD. 6-MP metabolite monitoring is advantageous for identifying causes of failure to respond to 6-MP therapy. For example, 6-MP metabolite levels can be used to indicate an inadequate dose or an excessive dose that could lead to toxicity. Additionally, monitoring of 6-MP metabolites can also be used to indicate non-compliance with a therapeutic regimen.

The methods of the invention directed to determining whether a patient has a minimal therapeutic level of a 6-MP metabolite are useful for indicating to the clinician a need to monitor a patient for therapeutic efficacy and to adjust the 6-MP drug dose, as desired. For example, in a patient having less than a minimal therapeutic level of a 6-MP metabolite such as 6-TG and who also presents as unresponsive to 6-MP drug therapy or having poor responsiveness to 6-MP drug therapy as measured by minimal or no effect on a sign or symptom of the disease being treated, one skilled in the art can determine that the dosage of a 6-MP drug should be increased. However, if it is determined that a patient has less than a predetermined minimal therapeutic level of a 6-MP metabolite but is responsive to 6-MP therapy, the current dose of 6-MP drug can be maintained. Based on measuring 6-MP metabolite levels and assessing the responsiveness of the patient to 6-MP therapy, one skilled in the art can determine whether a 6-MP drug dose should be maintained, increased, or decreased.

Although 6-MP drugs such as 6-MP and azathioprine can be used for effective treatment of an immune-mediated GI disorder, including IBDs such as Crohn's disease or ulcerative colitis, administration of such drugs can be associated with toxic side effects. Toxicities associated with 6-MP drug administration include pancreatitis, bone marrow depression, allergic reactions and drug hepatitis as well as neoplasms and infectious complications (Present et al., Annals Int. Med. 111:641-649 (1989); Cuffari et al., *supra,* 1996a). As disclosed herein, various toxicities associated with 6-MP drug treatment, including hepatic toxicity, pancreatic toxicity and hematologic toxicity, correlate with the level of 6-MP metabolites in a subject administered a. 6-MP drug (see Examples I and II).

Thus, the present invention also provides an in vitro method of reducing toxicity associated with treatment of an immune-mediated GI disorder by administration of a 6-MP drug. The method of the invention for reducing toxicity associated with 6-MP drug treatment includes determining a level of a 6-MP metabolite in a sample from a subject having an immune-mediated GI disorder, where a level of the 6-MP metabolite greater than a predetermined toxic level of the 6-MP metabolite indicates a need to decrease the amount of 6-MP drug subsequently administered to the subject, thereby reducing toxicity associated with 6-MP drug treatment of the immune-mediated GI disorder. In a method of the invention, the 6-MP metabolite can be, for example, 6-TG and the predetermined toxic level of 6-TG can correspond, for example, to a level of about 400 pmol per 8x10⁸ red blood cells. Where the elevated 6-MP metabolite is 6-TG, the toxicity associated with 6-MP treatment can be, for example, hematologic toxicity, including leukopenia or bone marrow suppression. The 6-MP metabolite also can be a metabolite such as 6-MMP, and the predetermined toxic level of 6-MMP can correspond, for example, to a level of about 7000 pmol per 8x10⁸ red blood cells. Where the elevated 6-MP metabolite is 6-MMP, the toxicity associated with 6-MP drug treatment can be, for example, hepatic toxicity.

As disclosed herein, the level of a 6-MP metabolite can be determined in a subject treated with a 6-MP drug and compared to a predetermined toxic level of a 6-MP metabolite such as 6-TG or 6-MMP to adjust future 6-MP drug administration, thereby reducing toxicity in the subject. For example, as disclosed herein, levels of 6-TG above about 400 pmol/8x10⁸ RBC indicated that a patient was likely to experience toxicity, in particular hematologic toxicity such as leukopenia (see Examples I and II). Accordingly, a level of 6-TG above about 400 pmol/8x10⁸ RBC can be a predetermined toxic level of 6-TG, which indicates that the amount of 6-MP drug subsequently administered should be decreased.

It is understood that, when a patient is determined to have a level of a 6-MP metabolite such as 6-TG or 6-MMP higher than a predetermined toxic level, one skilled in the art can make a determination as to whether a 6-MP drug dose should be decreased. For example, if the level of a 6-MP metabolite such as 6-TG or 6-MMP is higher than a predetermined toxic level, one skilled in the art can monitor for toxic side effects by measuring one or more of the toxicities associated with 6-MP drug treatment, as disclosed herein. As disclosed herein, a level of 6-TG greater than about 400 pmol per 8x10⁸ RBCs was associated with increased risk of leukopenia or bone marrow suppression. Therefore, one skilled in the art can measure white blood cells (WBC) in a patient having levels of 6-TG higher than a predetermined toxic level to determine if the patient is exhibiting signs of reduced WBC counts. If such a patient exhibits signs of leukopenia or bone marrow suppression, the 6-MP drug dose can be reduced. However, if it is determined that a patient has levels of a 6-MP metabolite higher than a predetermined toxic level but does not exhibit signs of leukopenia or other 6-MP drug toxicities, one skilled in the art can determine that the current 6-MP drug dose can be maintained. Based on measuring 6-MP metabolite levels and determining signs or symptoms of toxicities associated with 6-MP drug treatment, one skilled in the art can determine whether a 6-MP drug dose should be maintained or decreased. As such, a level of a 6-MP metabolite higher than a predetermined toxic level can indicate a need to measure a toxicity associated with 6-MP drug treatment such as measuring WBCs or any of the other signs or symptoms of toxicities associated with 6-MP drug treatment to determine if the 6-MP drug dose should be adjusted.

Furthermore, it is understood that, when decreasing the 6-MP drug dose, one skilled in the art will know or can readily determine whether the 6-MP drug dose should be decreased to a lower dose or whether 6-MP drug administration should be stopped for some period of time, or terminated. For example, if the clinician determines that 6-MP drug therapy should be stopped for some period of time due to levels of a 6-MP metabolite exceeding a level predetermined to be toxic, the levels of 6-MP metabolites can be monitored after stopping 6-MP drug therapy until the level of the toxic 6-MP metabolite returns to a safe, non-toxic level. At that time, the clinician can resume 6-MP drug therapy, if desired.

The methods of the invention for reducing toxicity associated with 6-MP drug treatment of a disease involve comparing a level of a 6-MP metabolite to a predetermined toxic level of a 6-MP metabolite. In general, a "predetermined toxic level" of a 6-MP metabolite means a level of a 6-MP metabolite that has been correlated with one or more toxicities associated with 6-MP drug treatment. As disclosed herein, a predetermined toxic level of 6-TG can be about 400 pmol per 8x10⁸ RBC. A predetermined toxic level of 6-TG also can be about 350 pmol per 8x10⁸ RBC; 370 pmol per 8x10⁸ RBC; 390 pmol per 8x10⁸ RBC; 425 pmol per 8x10⁸ RBC; or 450 pmol per 8x10⁸ RBC. It is understood that the predetermined toxic levels of 6-TG disclosed herein are useful for treating immune-mediated GI disorders, including IBD, as well as non-IBD autoimmune diseases.

Another 6-MP metabolite useful for predicting the likelihood of toxicity is 6-methyl-mercaptopurine (6-MMP). As disclosed herein, a level of greater than about 7000 pmol 6-MMP/8x10⁸ in patients administered a 6-MP drug was associated with toxicity, in particular hepatotoxicity (see Examples I and II). These results indicate that the level of 6-MMP can be used to predict toxicity in a patient treated with a 6-MP drug. As disclosed herein, a predetermined toxic level of 6-MMP can be about 7000 pmol per 8x10⁸ RBC. A predetermined toxic level of 6-MMP also.can be about 6000 pmol per 8x10⁸ RBC; 6500 pmol per 8x10⁸ RBC; 7500 pmol per 8x10⁸ RBC; or 8000 pmol per 8x10⁸ RBC. Furthermore, a predetermined toxic level of 6-MMP can also be about 5500 pmol per 8x10⁸ RBC or about 5700 pmol per 8x10⁸ RBC (see Example II). It is understood that the predetermined toxic levels of 6-MMP disclosed herein are useful for treating immune-mediated GI disorders, including IBD, as well as non-IBD autoimmune diseases. According to a method of the invention, if the level of 6-MMP is above a predetermined toxic level, the subsequent administration of a 6-MP drug can be decreased to minimize toxicity.

Further provided by the invention is an in vitro method of optimizing therapeutic efficacy and reducing toxicity associated with treatment of an immune-mediated GI disorder such as IBD by administration of a 6-MP drug. The method includes the steps of determining a level of 6-TG in a sample from a subject having an immune-medicated GI disorder; and determining a level of 6-MMP in the subject having the immune-mediated GI disorder, where a level of 6-TG less than a predetermined minimal therapeutic level indicates a need to increase the amount of 6-MP drug subsequently administered to the subject, thereby increasing therapeutic efficacy; where a level of 6-TG greater than a predetermined toxic level of 6-TG indicates a need to decrease the amount of 6-MP drug subsequently administered to the subject, thereby reducing toxicity associated with 6-MP treatment of the immune-mediated GI disorder; and where a level of 6-MMP greater than a predetermined toxic level of 6-MMP indicates a need to decrease the amount of 6-MP drug subsequently administered to the subject, thereby reducing toxicity associated with 6-MP drug treatment of the immune-mediated GI disorder.

In such a method of optimizing therapeutic efficacy and reducing toxicity associated with 6-MP drug treatment of an immune-mediated GI disorder such as IBD, the predetermined minimal therapeutic level of 6-TG can be, for example, a level corresponding to about 230 pmol per 8x10⁸ red blood cells; the predetermined toxic level of 6-TG can be, for example, a level corresponding to about 400 pmol per 8x10⁸ red blood cells; and the predetermined toxic level of 6-MMP can be, for example, a level corresponding to about 7000 pmol per 8x10⁸ red blood cells. In a method of the invention, the subject having an immune-mediated GI disorder such as IBD can be, for example, a pediatric subject. The level of 6-TG and 6-MMP each can be conveniently determined, for example, in red blood cells using HPLC.

The methods of the invention are useful for optimizing the amount of a 6-MP drug to be administered to a patient with an immune-mediated GI disorder such as IBD. By measuring the levels of 6-MP metabolites such as 6-MMP and 6-TG, one skilled in the art can determine the 6-MP drug dosage that will result in optimized therapeutic efficacy and reduced toxicity when administered to a patient.

As disclosed herein, gender and age differences were observed in pediatric patients treated with 6-MP drug therapy (see Example III). Very little change in 6-MP metabolite levels of 6-TG and 6-MMP was seen for girls who had gone through puberty (older than age 12). However, boys who had gone through puberty (older than age 14) had a marked decrease in the level of 6-MMP, suggesting that hormonal changes occurring during puberty can affect the metabolism of a 6-MP drug. Therefore, the methods of the invention can additionally be used to monitor 6-MP metabolite levels in adolescents, particularly those going through puberty, in order to optimize therapeutic efficacy or minimize toxic side effects associated with 6-MP therapy.

As disclosed herein, TPMT genotyping is useful for predicting the effectiveness of 6-MP therapy in an IBD patient (see Example IV). Heterozygote patients are expected to have lower TPMT activity and should therefore be monitored for high levels of 6-TG for possible toxic levels associated with leukopenia or bone marrow suppression. 6-MP drug doses can be reduced accordingly. Wild type homozygous patients are expected to have higher TPMT activity and should therefore be monitored to maintain an effective therapeutic level of 6-TG and to determine if patients develop toxic levels of 6-MMP. Homozygous patients deficient in TPMT activity can be treated with lower doses of a 6-MP drug provided that patients are closely monitored for toxicity such as leukopenia. Therefore, TPMT genotyping can be used to predict patient responsiveness to and potential toxicities associated with 6-MP drug therapy. Furthermore, TPMT genotyping can be combined with other methods of the invention to both determine TPMT genotype and to monitor 6-MP metabolites. TPMT genotyping can be particularly valuable when determining a starting dose of 6-MP drug therapy but can also be useful when adjusting 6-MP drug doses after therapy has begun.

The invention additionally provides an in vitro method of optimizing therapeutic efficacy of treatment of a non-IBD autoimmune disease by administration of a 6-MP drug. The method includes determining a level of 6-thioguanine (6-TG) in a sample from a subject having a non-IBD autoimmune disease, where a level of 6-TG less than a minimal therapeutic level indicates a need to increase the amount of 6-MP drug subsequently administered to the subject and where a level of 6-TG greater than a predetermined toxic level indicates a need to decrease the amount of 6-MP drug subsequently administered to the subject. The level of 6-MMP can also be monitored in a patient having a non-IBD autoimmune disease to determine if the level is higher than a predetermined toxic level of 6-MMP.

The methods of the invention can be used to optimize therapeutic efficacy of 6-MP drug treatment of a non-IBD autoimmune disease. Such a non-IBD autoimmune disease can be any non-IBD autoimmune disease treatable by a 6-MP drug such as 6-MP or azathioprine and, in particular, can be a disease such as rheumatoid arthritis, systemic lupus erythematosus, autoimmune hepatitis (chronic active hepatitis) or pemphigus vulgaris.

As used herein, the term "non-IBD autoimmune disease" means a disease resulting from an immune response against a self tissue or tissue component, including both self antibody responses and cell-mediated responses. The term non-IBD autoimmune disease encompasses organ-specific non-IBD autoimmune diseases, in which an autoimmune response is directed against a single tissue, including myasthenia gravis, vitiligo, Graves' disease, Hashimoto's disease, Addison's disease, autoimmune gastritis, and Type I diabetes mellitus. The term non-IBD autoimmune disease also encompasses non-organ specific autoimmune diseases, in which an autoimmune response is directed against a component present in several or many organs throughout the body. Non-organ specific autoimmune diseases include, for example, systemic lupus erythematosus, progressive systemic sclerosis and variants, polymyositis and dermatomyositis, and rheumatoid disease. Additional non-IBD autoimmune diseases include pernicious anemia, primary biliary cirrhosis, autoimmune thrombocytopenia, and Sjögren's syndrome. One skilled in the art understands that the methods of the invention can be applied to these or other non-IBD autoimmune diseases treatable by a 6-MP drug such as 6-MP or azathioprine, or other 6-MP drugs, as desired. Specifically excluded from the term "non-IBD autoimmune disease" are diseases resulting from a graft versus host response and inflammatory bowel diseases such as Crohn's disease or ulcerative colitis. The methods of the invention are also useful for optimizing the treatment of a non-immune-mediated GI disorder autoimmune disease. As used herein, the term "non-immune-mediated GI disorder autoimmune disease" is a non-IBD autoimmune disease and specifically excludes immune-mediated GI disorders.

The methods of the invention can be particularly useful for optimizing therapeutic efficacy of 6-MP drug treatment of rheumatoid arthritis. Rheumatoid arthritis is a chronic systemic disease primarily of the joints, usually polyarticular, marked by inflammatory changes in the synovial membranes and articular structures and by muscle atrophy and rarefaction of the bones.

The methods of the invention also can be particularly valuable for optimizing therapeutic efficacy of 6-MP drug treatment of lupus erythematosus and, in particular, systemic lupus erythematosus (SLE). Systemic lupus erythematosus is a chronic, remitting, relapsing inflammatory, and sometimes febrile multisystemic disorder of connective tissue. SLE can be.acute or insidious at onset and is characterized principally by involvement of the skin, joints, kidneys and serosal membranes.

Autoimmune hepatitis, also called chronic active hepatitis, also can be treated with a 6-MP drug and the dose optimized using the methods of the invention. Autoimmune hepatitis is a chronic inflammation of the liver occurring as a sequel to hepatitis B or non-A, non-B hepatitis and is characterized by infiltration of portal areas by plasma cells and macrophages, piecemeal necrosis, and fibrosis.

The methods of the invention also can be useful for optimizing the treatment of pemphigus vulgaris, the most common and severe form of pemphigus, which is a chronic, relapsing and sometimes fatal skin disease characterized clinically by the development of successive crops of vesicles and bullae and treated by azathioprine. This disorder is characterized histologically by acantholysis, and immunologically by serum autoantibodies against antigens in the intracellular zones of the epidermis.

The methods of the invention can also be used to optimize the therapeutic efficacy of 6-MP drug treatment of graft versus host disease, which can occur in transplant patients. Graft versus host disease occurs when a transplant patient has an immune reaction to the non-self transplant organ or tissue. The methods of the invention for optimizing the therapeutic efficacy of 6-MP drug treatment is particularly useful for treating heart, kidney and liver transplant recipients. The methods of the invention can be used to optimize therapeutic efficacy and/or minimize toxicity associated with 6-MP drug treatment of a transplant patient.

### EXAMPLE I

### 6-MERCAPTOPURINE METABOLITE LEVELS PREDICT CLINICAL EFFICACY AND DRUG TOXICITY IN PEDIATRIC IBD

This example describes measuring 6-MP metabolite levels and correlation with the response of IBD patients treated with a 6-MP drug.

The levels of the 6-MP metabolites 6-TG and 6-MMP were measured in IBD patients to whom 6-MP pro-drug was administered, and the relationship of 6-MP metabolites to clinical disease activity and drug toxicity was determined. Briefly, blood was sampled (n=89) prior to daily administration of 6-MP in 55 IBD patients (CD n=51, UC n=4) receiving 1-1.5 mg/kg/day over at least a 4 month period (≥ 4 mo.). When AZA was administered, a conversion factor of 2.07 was used to convert to the equivalent 6-MP dose. Twice as much AZA is administered relative to 6-MP to have an equivalent dose of 6-MP.

Erythrocyte 6-TG, 6-MMP and 6-MP thiobases were measured (pmol/8x10⁸ RBC) using reverse phase HPLC. Briefly, blood samples were collected in EDTA (ethylene diamine tetraacetic acid) as anticoagulant. Cells were centrifuged and washed three times with an equal volume of 0.9% saline. Washed packed cells were stored at -70°C until analysis was performed.

For acid hydrolysis, 500 µl deionized H₂O, 50 µl thawed red blood cells, 40 µl of the appropriate standard or control, 500 µl 3.0 N H₂SO₄, and 300 µl 10 mM dithiothreitol (DTT) was added to an 8 ml glass screw cap tube. The capped tubes were placed in a heating block preheated to 100°C and hydrolyzed. For 6-MMP, hydrolysis was carried out for 5 hours. For 6-MP and 6-TG, hydrolysis was carried out for 1 hour. After the incubation, tubes were cooled in a room temperature water bath. To tubes hydrolyzed for 5 hours (6-MMP), 400 µl 3.4 N NaOH and 1.0 ml 2 M Tris buffer, pH 9.0 was added. To tubes hydrolyzed for 1 hour (6-MP/6-TG), 450 µl 3.4 N NaOH and 500 µl 2M Tris base was added. A volume of 4 ml 0.03% phenylmercuric chloride in methylene chloride was added to each tube. The tubes were capped and lightly agitated on a bi-directional rotator (15 min for 6-MMP and 30 min for 6-MP/6-TG). The contents were transferred to a 15 ml polypropylene centrifuge tube and centrifuged at 3500 rpm for 3 min at 10°C. The aqueous phase (top layer) was discarded, and 3.0 ml of the organic phase (bottom layer) was transferred to a clean 15 ml polypropylene centrifuge tube. The analytes in the organic phase were back extracted by adding 225 µl 0.1 N HCl and lightly mixing on an orbital rotator for 5 min. Following vortexing for 30 seconds, the tubes were centrifuged at 3500 rpm for 3 min at 10°C.

For 6-MMP analysis, 50 µl analyte was analyzed on a C18 reverse phase column with the mobile phase containing 1 mM DTT, 2.078% triethylamine and 4% methanol, adjusted to pH 3.2 with concentrated H₃PO₄. For 6-MP and 6-TG analysis, 100 µl analyte was analyzed on a C18 reverse phase column using 0.1 M H₃PO₄ and 1 mM DTT in H₂O as the mobile phase.

Hepatic, pancreatic and hematological tests were obtained every 3 months. Clinical remission was defined as a Harvey Bradshaw Index < 5 in those patients off corticosteroids or weaned to a level of prednisone ≤ 0.4 mg/kg/od (administered every other day). Treatment failures were defined as non-responders (HBI > 5 or steroid dependence) or cessation of 6-MP due to side effects.

As shown in Table 1, a 6-TG level of >225 pmol per 8x10⁸ RBC was associated with remission. The median values shown in the tables represent pmol of the indicated 6-MP metabolite per 8x10⁸ RBC. Excessive 6-TG and 6-MMP levels were associated with leukopenia and hepatotoxicity, respectively. Negligible metabolite levels detected non-compliance as a cause of treatment failure in 2/31 cases. These results indicate that 6-MP metabolite levels predict both clinical responsiveness and drug-related toxicity.

**Table 1:**

| Group | n | median 6-TG | 6-TG >225 | median 6-MMP |
|---|---|---|---|---|
| Responders | 58 | 295 | 45/58 (78%) | 3094 |
| Non-responders | 31 | 184* | 8/31 (26%) | 2048 |
| Hepatic toxicity | 7 | 258 | 5/7 | 9211** |
| Pancreatic toxicity | 6 | 211 | 2/6 | 2342 |
| Hematologic toxicity | 6 | 414+ | 5/5 | 7042 |
| p Values | | *<0.001 | ‡0<0.001 | **<0.001 |
| | | +<0.03 | | |

These results demonstrate that determining levels of 6-MP metabolites is useful for predicting efficacy and toxicity of 6-MP drug therapy administered to IBD patients.

### EXAMPLE II

### 6-MERCAPTOPURINE METABOLITE LEVELS CORRELATE WITH OPTIMAL 6-MP THERAPY IN IBD PATIENTS

This example describes prospective examination of the correlation of 6-MP metabolite levels with therapeutic response to 6-MP drug therapy and 6-MP drug related toxicity in IBD patients treated with 6-MP.

To obtain additional statistical data on IBD patients treated with a 6-MP drug, additional patients and samples were analyzed and combined with the data obtained in Example I. Blood was sampled at least once in 92 IBD patients followed by the clinical investigators at Sainte-Justine Hospital IBD Center, Montreal, Canada, who were administered 6-MP drug therapy for at least 4 months. Patients were less than 19 years of age and were eligible for inclusion if receiving 6-MP or AZA for at least 4 months. Diagnoses of Crohn's disease (CD), ulcerative colitis (UC) or indeterminate colitis (IC) were established by standard clinical, radiological, histological and endoscopic criteria (Lennard-Jones, Scand. J. Gastroenterol Suppl. 170:2-6 (discussion 16-19)(1989)). Patients with leukopenia or with elevated serum hepatic or pancreatic enzyme activities prior to initiation of therapy were excluded from this study.

The dose of AZA/6-MP, determined by the treating physician, was stable for at least one month. Clinical information collected included: (a) Patient related data - gender, age at diagnosis, site of disease. (b) Disease activity - assessed at each visit corresponding to a 6-MP metabolite blood drawing, by calculating a validated index based on direct questioning, clinical examination and laboratory data. A modified Harvey Bradshaw Index (HBI)(Myren et al. Scand. J. Gastroenterol Suppl. 95:1-27 (1984)) was used for CD, Truelove & Witts' Index for UC (Truelove and Witts, Br. Med. J. 2:1041-1044 (1955)), or a detailed assessment of perianal disease and fistulae was used as previously described (Korelitz and Present, Dig. Dis. Sci. 30:58-64 (1985) (Hughes, Dis. Colon Rectum 35:928-932 (1992). (c) 6-MP related data - age at initiation, indication, dose (mg/kg/day). (d) Toxicity related data - complete blood count and differential, ALT, AST, billirubin, amylase and lipase, dose of 6-MP, timing of event relative to initiation of therapy, course of action taken and concurrent medications. Blood samples from 92 consenting patients, for analyzing 6-MP, 6-TG and 6-MMP moieties, were obtained prospectively during the study period (1995-1998). When possible, multiple samples were obtained from the same patient separated by an interval exceeding 3 months of the first specimen. The clinical investigators remained blinded to the 6-MP metabolite levels and the results of TPMT genotyping.

The 92 patients were pediatric patients, with 79 diagnosed as having CD, 8 diagnosed as having UC, and 5 diagnosed as having indeterminate colitis (CD or UC) (see Table 2). Seven patients were given AZA. The dosages of AZA were converted to 6-MP equivalents using a factor of 2.07 (Sandborne, Am. J. Gastroenterol. 91:423-433 (1996)).

**Table 2.**

| **Characteristics of Pediatric IBD Patients Treated With 6-MP/AZA** | |
|---|---|
| Gender (male-female) | 41:51 |
| Mean age at diagnosis (yr/range) | 11.5(1-18) |
| Disease classification and location | |
| Crohn's Disease (n) | 79 |
| Small Bowel (n) | 2 |
| Small Bowel + perianal +/or duodenal involvement (n) | 1 |
| Ileocolitis (n) | 33 |
| Ileocolitis + perianal +/or duodenal involvement (n) | 21 |
| Colitis alone (n) | 13 |
| Colitis + perianal +/or duodenal involvement (n) | 8 |
| Perianal disease alone (n) | 1 |
| Ulcerative Colitis (n) | 8 |
| Pancolitis (n) | 6 |
| Left sided (n) | 2 |
| Indeterminate Colitis (n) | 5 |
| Mean age at initiation of 6-MP/AZA (yr(range)) | 13(4-18.5) |
| Delay from diagnosis to start of 6-MP/AZA (yr (range)) | 1.5(0-7) |
| Primary indication for 6-MP/AZA use | |
| Steroid dependent (n) | 68 |
| Steroid refractory (n) | 17 |
| Fistulizing disease (n) | 7 |
| Mean 6-MP/AZA dose (mg/kg/day, (range)) | 1.25(0.4-2.4) |

The study population was divided into patients with a satisfactory clinical response and those who failed therapy. A clinical evaluation point was designated as a clinic visit or outpatient visit corresponding to each 6-MP metabolite measurement. Primary outcomes were based on each individual patient's initial indication for 6-MP. For those patients with chronic active or steroid dependent disease, response was defined by clinical remission based on a modified HBI score <4 for CD (Myren et al., *supra,* 1984) or according to the criteria for remission defined by Truelove and Witts criteria (Truelove and Witts, *supra*, 1955), in the absence of oral or parenteral corticosteroids. Complete fistula closure was considered a response for patients in whom fistulizing disease was the primary indication. Treatment failure applied to cases where the above stated therapeutic goals were not achieved. Response to 6-MP was defined by clinical remission (HBI <5, closed fistula) without corticosteroids. Disease activity and physical exam were ascertained at each clinic visit at which 6-MP metabolite levels were determined (clinical evaluation point). Hematological, pancreatic and hepatic laboratory parameters were evaluated simultaneously.

Among the single sampled patient group, 27 of 46 (59%) responded and 19 of 46 (41%) failed. In the multi-sampled patients, 20 of 46 (43%) had a response sustained at each clinical evaluation point, 11 (24%) never achieved a clinical response and 15 (33%) responded inconsistently over time. Among all 173 metabolite measurements, 103 (60%) corresponded to patients with a clinical response to therapy and 70 (40%) were in patients not responding at the designated clinical evaluation points (see Table 3).

Erythrocyte 6-TG and 6-MMP concentrations (pmol/8x10⁸ RBC) were measured by HPLC (Cuffari et al., *supra,* 1996a). Briefly, heparinized blood (5 ml) was obtained along with other routine blood work at the time of a scheduled clinic visit or hospitalization. RBC free thiobases, 6-MP, 6-TG and 6-MMP (pmol/8x10⁸ RBC) levels were determined by a reversed phase high performance liquid chromatography assay (Cuffari et al., *supra,* 1996a).

In an initial analysis, the induction and maintenance of remission was found to be optimal at 6-TG levels >230 pmol/8x10⁸ RBC. 78% of patients above a median 6-TG of 230 pmol/8x10⁸ RBC were responders (see Figure 3). These results indicate that a 6-TG value of 230 pmol/8x10⁸ RBC can be used to predict efficacy of drug treatment with a 6-MP drug such as 6-MP or azathioprine.

The results of the analysis of 6-MP metabolites in IBD patients are shown in Table 3. The number of samples corresponds to the number of different samples obtained from the 92 patients. Overall, higher median 6-TG levels were observed at points of clinical response vs non-response (312 vs 199; p<0.0001) (Table 3). Secondary analysis of all 92 patients tested at their initial clinical evaluation point revealed similar results (313 vs 209 at 53 points of response vs 39 non-response, p<0.004). 6-TG quartile analysis on all samples, in which values are determined at 25, 50 and 75% of the data set, revealed that the best probability of treatment response was not significantly increased until RBC 6-TG levels were greater than 235 pmol/8x10⁸ RBC (Figure 4A). Patients' 6-TG levels were evenly distributed within the third quartile, suggesting 235 pmol/8x10⁸ RBC as the true threshold point. Among patients in relapse, only 27% (20 of 70) of patients had 6-TG levels >235 pmol/8x10⁸ RBC (Figure 5). In contrast, 67% (69 of 103) of patients in remission, at times of response, had 6-TG levels > 235 pmol/8x10⁸ RBC (p <0.001). Presenting the data in an alternate manner useful clinically, the odds ratio of a therapeutic response for a 6-TG level higher than the cutoff of 235 pmol/8x10⁸ RBC was calculated to be 5.0 (95% CI, 2.6-9.7; p<0.001). Therefore, erythrocyte 6-TG concentrations were significantly and independently associated with therapeutic response to 6-MP drug therapy.

**Table 3.**

| **6-Mercaptopurine Metabolite Levels At Designated Clinical Evaluation Points In Relation To Therapeutic Efficacy In IBD.** | | |
|---|---|---|
| THERAPEUTIC EFFICACY | 6-TG LEVEL^{†} pmol/8x10⁸ RBC | 6-MMP LEVEL^{†} (pmol/8x10⁸ RBC) |
| RESPONSE (n=103) | 312 (49-1203) | 2683 (0-23 129) |
| FAILURE (n=70) | 199 (0-705) | 1739 (0-25 982) |
| p value | <0.0001 | 0.37 |

| | | |
|---|---|---|
| †Median (range). | | |

6-MMP levels did not correlate with disease activity, nor did quartile analysis demonstrate a specific level that corresponded with outcome in the primary (Figure 4B) or secondary analyses (2683 vs 1420 for response and non-response). The theoretically competitive enzymatic pathways determining concentrations of 6-TG and 6-MMP suggest a negative correlation between these two metabolites. Nevertheless, a positive correlation between 6-TG and 6-MMP was observed (r=0.21, p <0.007). 6-MP moieties were undetected in most samples, indicating that 6-MP and 6-thioinosine 5' monophosphate had been entirely biotransformed.

Statistical analysis was performed using SAS computer software (Version 6.10; SAS Institute, Inc., Cary, NC, 1994). Quantitative variables are described as medians, with the range indicated in parentheses. Normality was tested for all quantitative variables. Both parametric and nonparametric statistic tests were used, accordingly based on the distribution of variables in order to evaluate correlation between two variables and to test mean/median differences of quantitative variables between two groups. Statistical differences in proportions were evaluated by using the χ² method or Fisher's Exact tests, when appropriate.

To evaluate the relationship between therapeutic response and metabolite levels, linear logistic regression was performed by the maximum likelihood method. The dependent variable was therapeutic response or failure for an individual, either at one evaluation point or consistently over time. Independent variables were the patient characteristics detailed in Table 2, the 6-MP dose and metabolite levels, as well as TMPT genotype. In this analysis, for individuals with blood drawn for metabolite levels at more than one evaluation point, mean values were used and cases with mixed clinical responses (n=16) were excluded. However, they were used to identify therapeutic response-associated factors within each individual with paired tests (response vs failure).

Logistic regression analysis revealed that 6-TG levels were significantly associated with therapeutic response, independent of other confounding factors potentially affecting prognosis (p=0.002). For each of the 15 patients inconsistently responsive over time, the values of 6-TG, 6-MMP, dose and duration of therapy were compared between points of clinical response and of non-response. Again, the 6-TG level was the only statistically significant variable determining therapeutic response (p<0.02). Unsuspected non-compliance was confirmed in 3 patients by documenting metabolite levels below the limits of detection.

A meta-analysis reported a beneficial effect of increasing 6-MP dose in treating quiescent CD, although not for active CD (Pearson et al., Ann. Intern. Med. 123:132-142 (1995)). In the present study, the median dose of 6-MP was not statistically different in relation to therapeutic response (1.25 and 1.25 mg/kg/day, for response and treatment failures, respectively). Similarly, there was no difference in dose when analyzed at the time of the first clinical assessment alone (1.2 vs 1.1 mg/kg/day). Quartile analysis demonstrated that the frequency of remission was not significantly different across the dose range (Figure 6). Since 6-TG levels correlated with response, the relationship between 6-MP dose and metabolite level was examined. A nonlinear, very weakly positive correlation was observed between dose and 6-TG (r=0.0009), and 6-MMP (r=0.32) levels.

In addition to the lack of, or delay in, the therapeutic response to 6-MP, drug related toxicity has led to the underutilization of this class of immunosuppressive therapy in IBD. Toxicity in the IBD patients treated with a 6-MP drug was therefore evaluated. Toxicity was measured essentially as described previously (Cuffari et al., *supra*, 1996a). Toxicity was defined as: (a) hematological - leukocytes <4000/mm³; (b) hepatic - aspartate aminotransferase (AST) or alanine aminotransferase (ALT) >2x upper limit of normal; (c) pancreatic - amylase or lipase >2x upper limit of normal (Goldstein et al., Gastroenterology 114:A4042 (1998); Kirschner, Gastroenterology 115:813-821 (1998)). Thirty six patients (36 of 92)(39%) experienced an adverse event attributable to 6-MP drug therapy. As a measure of hepatotoxicity, elevated transaminases, measured as the serum level of ALT or AST (ALT, AST; exceeding or greater than 2x upper limit of normal) occurred in 16 (17%) patients. Pancreatic toxicity, measured as increased amylase and lipase levels (lipase/amylase >2x N), was observed in 7 cases (8%). However, only one patient developed clinically overt pancreatitis. Another experienced hepatic and pancreatic toxicity simultaneously. In all but 1 of 16 events, reduction of the dose of 6-MP was associated with normalization of transaminases within weeks. In the latter case, extensive investigations revealed steatohepatitis.

High 6-MMP levels correlated significantly with hepatotoxicity (5463 with hepatotoxicity versus 2177 without hepatotoxicity; p=0.04). As shown in Figure 7A, median 6-MMP levels were significantly higher at the time of a hepatotoxic event (n=16). Although hepatotoxicity was observed at low 6-MMP levels, the risk increased three-fold (18 vs 6%) at 6-MMP levels above the third quartile (>5,700). 6-TG levels did not affect hepatotoxicity, nor did the 6-MP dose.

In regard to hematological effects, leukopenia occurred in 13 individuals (14%). The median 6-TG level during leukopenic events in 12 individuals was 286, as compared to 232 without leukopenia (p=0.06). Two-thirds of leukopenic events occurred during remission, representing only 8% of all therapeutic responses. Significantly higher 6-TG levels were observed in these patients in remission (mean value of 490 in patients with leukopenia versus mean value of 323 without leukopenia; p <0.04; median values were 342 versus 307, respectively). The majority (92%) of clinical responses thus occurred in the absence of leukopenia. 6-TG levels correlated significantly with the presence of leukopenia at points of therapeutic response (Figure 7B). Although 4 of 70 (6%) leukopenic events occurred during points of treatment failure, the 6-TG levels were lower (198 (range 101-215)). Neither the 6-MP dose nor 6-MMP levels influenced leukopenia.

On average, hematological toxicity occurred later in the course of 6-MP therapy compared to hepatic or pancreatic complications (means of 1.5, vs 0.4, and 0.6 years, respectively). Permanent discontinuation of 6-MP as a result of myelosuppression was deemed necessary in 1 of these patients (6-TG=793). Leukopenia did not correlate with therapeutic efficacy. However, those patients who do develop leukopenia have a higher 6-TG level than the rest of the responder group. These results indicate that 6-TG levels should be monitored to avoid potential clinical bone marrow suppression in responder patients who have high levels of 6-TG. No correlation between 6-MP dose or metabolite levels and pancreatic toxicity was observed.

Kinetic studies have demonstrated that derivatives of benzoic acid such as sulfasalazine and 5-aminosalicylic acid (5-ASA)(ASACOL™), its active metabolite, inhibit TPMT activity *in vitro* (Szumlanski et al., Br. J. Clin. Pharmacol. 39:456-459 (1995). In this study, 48 of 92 (52%) of patients were treated with 5-ASA concomitantly (mean 45 mg/kg/day). Of 173 metabolite levels measured, 77 coincided with 5-ASA use. No effect of concurrent 5-ASA use was observed on either therapeutic response, 6-TG and 6-MMP determination and levels, or drug related toxicity.

These results demonstrate a significant correlation between erythrocyte 6-TG levels and the therapeutic response to 6-MP drug treatment in IBD patients. The induction and maintenance of remission was found to be optimal at 6-TG levels greater than about 230 pmol/8x10⁸ RBC, for example about 235 pmol/8x10⁸ RBC. Therefore, monitoring 6-MP metabolite levels, in particular 6-TG, is useful for determining that a therapeutically effective concentration of 6-MP metabolites is maintained while treating IBD patients with a 6-MP drug. Monitoring 6-MP metabolite levels, in particular 6-TG and 6-MMP, is also useful for minimizing 6-MP drug related toxicity.

### EXAMPLE III

### GENDER AND AGE DIFFERENCES IN METABOLISM OF A 6-MP DRUG

This example describes gender and age differences observed in pediatric patients treated with 6-MP drug therapy.

Pediatric IBD patients undergoing 6-MP drug therapy were assessed for levels of 6-MP metabolites. These patients were wild type for TPMT. Patients were assessed based on gender and age as it relates to puberty. Puberty is established at 12 years of age in girls and 14 years of age in boys.

As shown in Table 4, the 6-MMP values are much lower in boys after puberty (greater than 14 years). Since the total amount of thiometabolites is lower, this indicates that either lower doses of 6-MP are used or that there is a difference in the bioavailability of 6-MP after puberty in males.

**Table 4:**

| 6-MP Metabolite Levels in Pediatric IBD Patients | | | | |
|---|---|---|---|---|
| | Number Observed | 6-TG level (pmol per 8x10⁸ RBC) | 6-MMP level (pmol per 8x10⁸ RBC) | Ratio 6-MMP/6-TG |
| Girls (0-12y) | 39 | 182.2 | 3447.0 | 18.52 |
| Girls (>12y) | 116 | 217.2 | 3304.5 | 12.55 |
| Boys (0-14y) | 51 | 235.0 | 3681.6 | 17.24 |
| Boys (>14y) | 104 | 222.5 | 1662.3* | 6.78* |

| | | | | |
|---|---|---|---|---|
| *p<0.001 | | | | |

These results demonstrate that gender and age can affect the metabolism of 6-MP in pediatric IBD patients undergoing 6-MP drug therapy.

### EXAMPLE IV

### THIOPURINE METHYLTRANSFERASE (TPMT) GENOTYPING AND RESPONSIVENESS TO 6-MP DRUG THERAPY

This example describes TPMT genotyping of IBD patients treated with 6-MP drug therapy.

The genotype of TPMT was determined in IBD patients that were responders and non responders. Genotyping of TPMT was measured essentially as described previously (Baccichet et al., Leuk. Res. 21:817-823 (1997); Zietkiewicz et al., Gene 205:161-171 (1997)). All individuals were genotyped for two polymorphisms in TPMT transitions at position 460 (G460A, Ala-to-Thr) and at position 719 (A719G, Tyr-to-Cys) using a PCR-allele specific oligonucleotide (ASO) hybridization assay (Szumlanski et al., DNA Cell Biol. 15:17-30 (1996); Tai et al., Am. J. Hum. Genet. 58:694-702 (1996); Zietkiewicz et al., Gene 205:161-171 (1997)). These mutations were used to define three distinct alleles: TMPT 3*A (both G460A and A719G), TMPT 3*B (only G460A) and TMPT 3*C (only A719G). The data shown in Table 5 indicate that patients heterozygous for the TPMT mutation had significantly higher 6-TG levels compared to those patients without the mutation. All heterozygote patients were responders to 6-MP.

**Table 5:**

| TPMT Genotyping of IBD Patients | | |
|---|---|---|
| | Heterozygote | Normal |
| Responder | 100% | 55% |
| Non responder | 0% | 45% |
| mean 6-TG | 589* | 247 |

| | | |
|---|---|---|
| p value *less than 0.0001 | | |

Among the patients examined, TPMT genotyping revealed that 8 (9%) of patients were heterozygous for the TPMT allele (TPMT^{H}/TPMT^{L}). No homozygous TPMT deficient patients were detected. All heterozygotes, compared to 39 of 84 (46%) of patients homozygous for normal alleles, achieved a clinical response (single and multisampled) on 6-MP and had 6-TG levels >230 pmol/8x10⁸ RBC. Thirty of the remaining 45 never responded and 15 were mixed responders. Higher 6-TG levels were found among heterozygotes (Figure 8). However, 6-MMP levels were comparable among heterozygotes and normals (1115 vs 2231 pmol/8x10⁸ RBC). The median dose of. 6-MP was not significantly different in relation to genotype (1.2 and 1.1 mg/kg/day, for normals and heterozygotes, respectively). Only 1 of 13 patients with leukopenia was heterozygous for the TPMT allele. Overall, 35 of 36 patients with drug related toxicity had a normal TPMT genotype.

These results demonstrate that TPMT genotyping is useful for predicting the effectiveness of 6-MP therapy in an IBD patient. Heterozygote patients are expected to have lower TPMT activity and should therefore be monitored for high levels of 6-TG for possible toxic levels associated with leukopenia or bone marrow suppression. 6-MP drug doses can be reduced accordingly. Wild type homozygous patients are expected to have higher TPMT activity and should therefore be monitored to maintain an effective therapeutic level of 6-TG and to determine if patients develop toxic levels of 6-MMP. Homozygous patients deficient in TPMT activity can be treated with lower doses of a 6-MP drug provided that patients are closely monitored for toxicity such as leukopenia.

## Claims

1. An *in vitro* method for determining efficacy of treatment of a subject having an immune-mediated gastrointestinal disorder or a non-inflammatory bowel disease (non-IBD) autoimmune disease by administration of a 6-mercaptopurine drug, comprising
determining *in vitro* a level of 6-thioguanine in a sample from said subject having said immune-mediated gastrointestinal disorder or said non-inflammatory bowel disease (non-IBD) autoimmune disease,
wherein said treatment is considered efficient if the level of 6-thioguanine is in the range of about 230 pmol per 8x10⁸ red blood cells to about 400 pmol per 8x10⁸ red blood cells.

2. The method of claim 1, wherein said 6-thioguanine level is about 240, 250, 260, 280 and 300 pmol per 8x10⁸ red blood cells.

3. The method of claim 1 or 2, wherein said method is useful for optimizing and/or monitoring efficacy of said treatment.

4. An *in vitro* method for determining toxicity of treatment of a subject having an immune-mediated gastrointestinal disorder or a non-inflammatory bowel disease (non-IBD) autoimmune disease by administration of a 6-mercaptopurine drug, comprising the steps of
(a) determining *in vitro* a level of 6-thioguanine in a sample from said subject having said immune-mediated gastrointestinal disorder or said non-inflammatory bowel disease(non-IBD) autoimmune disease, and/or
(b) determining *in vitro* a level of 6-methyl-mercaptopurine in a sample from said subject having said immune-mediated gastrointestinal disorder or said non-inflammatory bowel disease (non-IBD) autoimmune disease,
wherein the 6-mercaptopurine drug treatment is considered toxic if the level of 6-thioguanine is above about 350, 370, 390, 400, 425 or 450 pmol per 8x10⁸ red blood cells and/or the level of 6-methyl-mercaptopurine is above about 5500, 5700, 6000, 6500, 7000, 7500 or 8000 pmol per 8x10⁸ red blood cells.

5. The method of claim 4, wherein said method is useful for reducing and/or monitoring toxicity of said treatment.

6. An *in vitro* method for determining efficacy and toxicity of treatment of a subject having an immune-mediated gastrointestinal disorder or a non-inflammatory bowel disease (non-IBD) autoimmune disease by administration of a 6-mercaptopurine drug, comprising the steps of
(a) determining *in vitro* a level of 6-thioguanine in a sample from said subject having said immune-mediated gastrointestinal disorder or said non-inflammatory bowel disease (non-IBD) autoimmune disease, and/or
(b) determining *in vitro* a level of 6-methyl-mercaptopurine in a sample from said subject having said immune-mediated gastrointestinal disorder or said non-inflammatory bowel disease (non-IBD) autoimmune disease,
wherein said treatment is considered efficient if the level of 6-thioguanine is in the range of about 230 pmol per 8x10⁸ red blood cells to about 400 pmol per 8x10⁸ red blood cells and wherein said treatment is considered toxic if the level of 6-thioguanine is above about 350, 370, 390, 400, 425 or 450 pmol per 8x10⁸ red blood cells and/or the level of 6-methyl-mercaptopurine is above about 5500, 5700, 6000, 6500, 7000, 7500 or 8000 pmol per 8x10⁸ red blood cells.

7. The method of claim 6, wherein said 6-thioguanine level is about 240, 250, 260, 280 and 300 pmol per 8x10⁸ red blood cells.

8. The method of claim 6 or 7, wherein said method is useful for optimizing and/or monitoring efficacy and reducing and/or monitoring toxicity of said treatment.

9. The method of claims 4 to 8, wherein said level of 6-methyl-mercaptopurine is above about 7000 pmol per 8x10⁸ red blood cells.

10. The method of claims 1 to 9, wherein said immune-mediated gastrointestinal disorder is selected from the group consisting of lymphocytic colitis, microscopic colitis, collagenous colitis, autoimmune enteropathy, allergic gastrointestinal disease and eosinophilic gastrointestinal disease.

11. The method of claims 1 to 10, wherein said level of 6-thioguanine is determined in red blood cells.

12. The method of claims 1 to 11, wherein said level is determined using high pressure liquid chromatography.

## Patentansprüche

1. In vitro-Methode zur Bestimmung der Effizienz einer Behandlung eines Subjekts, das eine immun-vermittelte gastrointestinale Störung oder eine nicht-inflammatorische Darmerkrankung (nicht-IBD) Autoimmunerkrankung hat, durch Verabreichung eines 6-Mercaptopurin-Wirkstoffs, umfassend
Bestimmen eines 6-Thioguanin-Spiegels in einer Probe des Subjekts, das die immun-vermittelte gastrointestinale Störung oder die nicht-inflammatorische Darmerkrankung (nicht-IBD) Autoimmunerkrankung hat, *in vitro*
wobei die Behandlung als effizient angesehen wird, wenn der 6-Thioguanin-Spiegel im Bereich von etwa 230 pmol pro 8x10⁸ rote Blutzellen bis etwa 400 pmol pro 8x10⁸ rote Blutzellen liegt.

2. Methode nach Anspruch 1, bei der der 6-Thioguanin-Spiegel etwa 240, 250, 260, 280, und 300 pmol pro 8x10⁸ rote Blutzellen beträgt.

3. Methode nach Anspruch 1 oder 2, wobei die Methode nützlich ist, um die Behandlung zu optimieren und/oder deren Effizienz zu überwachen.

4. *In vitro*-Methode zur Bestimmung der Toxizität einer Behandlung eines Subjekts, das eine immun-vermittelte gastrointestinale Störung oder eine nicht-inflammatorische Darmerkrankung (nicht-IBD) Autoimmunerkrankung hat, durch Verabreichung eines 6-Mercaptopurin-Wirkstoffs, umfassend die Schritte
(a) Bestimmen eines 6-Thioguanin-Spiegels in einer Probe des Subjekts, das die immun-vermittelte gastrointestinale Störung oder die nicht-inflammatorische Darmerkrankung (nicht-IBD) Autoimmunerkrankung hat, *in vitro* und/oder
(b) Bestimmen eines 6-Methylmercaptopurin-Spiegels in einer Probe des Subjekts, das die immun-vermittelte gastrointestinale Störung oder die nicht-inflammatorische Darmerkrankung (nicht-IBD) Autoimmunerkrankung hat, *in vitro,*
wobei die Behandlung mit dem 6-Mercaptopurin-Wirkstoff als toxisch angesehen wird, wenn der 6-Thioguanin-Spiegel oberhalb von etwa 350, 370, 390, 400, 425 oder 450 pmol pro 8x10⁸ rote Blutzellen und/oder der 6-Mercaptopurin-Spielgel oberhalb von etwa 5500, 5700, 6000, 6500, 7000, 7500 oder 8000 pmol pro 8x10⁸ rote Blutzellen liegt.

5. Methode nach Anspruch 4, wobei die Methode nützlich ist, um die Toxizität der Behandlung zu reduzieren und/oder zu überwachen.

6. In vitro-Methode zur Bestimmung der Effizienz und Toxizität einer Behandlung eines Subjekts, das eine immun-vermittelte gastrointestinale Störung oder eine nicht-inflammatorische Darmerkrankung (nicht-IBD) Autoimmunerkrankung hat, durch Verabreichung eines 6-Mercaptopurin-Wirkstoffs, umfassend die Schritte
(a) Bestimmen eines 6-Thioguanin-Spiegels in einer Probe des Subjekts, das die immun-vermittelte gastrointestinale Störung oder die nicht-inflammatorische Darmerkrankung (nicht-IBD) Autoimmunerkrankung hat, *in vitro* und/oder
(b) Bestimmen eines 6-Methylmercaptopurin-Spiegels in einer Probe des Subjekts, das die immun-vermittelte gastrointestinale Störung oder die nicht-inflammatorische Darmerkrankung (nicht-IBD) Autoimmunerkrankung hat, *in vitro*,
wobei die Behandlung als effizient angesehen wird, wenn der 6-Thioguanin-Spiegel im Bereich von etwa 230 pmol pro 8x10⁸ rote Blutzellen bis etwa 400 pmol pro 8x10⁸ rote Blutzellen liegt und wobei die Behandlung als toxisch angesehen wird, wenn der 6-Thioguanin-Spiegel oberhalb von etwa 350, 370, 390, 400, 425 oder 450 pmol pro 8x10⁸ rote Blutzellen und/oder der 6-Mercaptopurin-Spielgel oberhalb von etwa 5500, 5700, 6000, 6500, 7000, 7500 oder 8000 pmol pro 8x10⁸ rote Blutzellen liegt.

7. Methode nach Anspruch 6, bei der der 6-Thioguanin-Spiegel etwa 240, 250, 260, 280, und 300 pmol pro 8x10⁸ rote Blutzellen beträgt.

8. Methode nach Anspruch 6 oder 7, wobei die Methode nützlich ist, um die Behandlung zu optimieren und/oder deren Effizienz zu überwachen und die Toxizität der Behandlung zu reduzieren und/oder zu überwachen.

9. Methode nach den Ansprüchen 4 bis 8, bei der der 6-Methylmercaptopurin-Spiegel oberhalb von etwa 7000 pmol pro 8x10⁸ rote Blutzellen beträgt.

10. Methode nach den Ansprüchen 1 bis 9, bei der die immun-vermittelte gastrointestinale Störung aus der Gruppe bestehend aus lymphocytischer Colitis, Mikroskopischer Colitis, kollagenöser Colitis, autoimmuner Enteropathie, allergischer gastrointestinaler Erkrankung und eosinophiler gastrointestinaler Erkrankung ausgewählt ist.

11. Methode nach den Ansprüchen 1 bis 10, bei der man den 6-Thioguanin-Spiegel in roten Blutzellen bestimmt.

12. Methode nach den Ansprüchen 1 bis 11, bei der man den Spiegel unter Verwendung von Hochdruckflüssigkeitschromatographie bestimmt.

## Revendications

1. Procédé *in vitro* pour déterminer l'efficacité du traitement d'un sujet ayant un trouble gastro-intestinal à médiation immune ou une maladie auto-immune qui n'est pas une maladie inflammatoire de l'intestin (non-IBD) par administration d'un médicament à la 6-mercaptopurine, comprenant :
la détermination *in vitro* d'un niveau de 6-thioguanine dans un échantillon dudit sujet ayant ledit trouble gastro-intestinal à médiation immune ou ladite maladie auto-immune qui n'est pas une maladie inflammatoire de l'intestin (non-IBD)
dans lequel ledit traitement est considéré comme efficace si le niveau de 6-thioguanine est dans la plage d'environ 230 picomoles pour 8x10⁸ globules rouges à environ 400 picomoles pour 8x10⁸ globules rouges.

2. Procédé selon la revendication 1, dans lequel ledit niveau de 6-thioguanine est d'environ 240, 250, 260, 280 et 300 picomoles pour 8x10⁸ globules rouges.

3. Procédé selon la revendication 1 ou 2, dans lequel ledit procédé est utile pour optimiser et/ou surveiller l'efficacité dudit traitement.

4. Procédé *in vitro* pour déterminer la toxicité du traitement d'un sujet ayant un trouble gastro-intestinal à médiation immune ou une maladie auto-immune qui n'est pas une maladie inflammatoire de l'intestin (non-IBD) par administration d'un médicament à la 6-mercaptopurine comprenant les étapes consistant à :
(a) déterminer *in vitro* un niveau de 6-thioguanine dans un échantillon dudit sujet ayant ledit trouble gastro-intestinal à médiation immune ou ladite maladie auto-immune qui n'est pas une maladie inflammatoire de l'intestin (non-IBD), et/ou
(b) déterminer *in vitro* un niveau de 6-méthyl-mercaptopurine dans un échantillon dudit sujet ayant ledit trouble gastro-intestinal à médiation immune ou ladite maladie auto-immune qui n'est pas une maladie inflammatoire de l'intestin (non-IBD),
dans lequel le traitement médicamenteux à la 6-mercaptopurine est considéré comme toxique si le niveau de 6-thioguanine est supérieur à environ 350, 370, 390, 400, 425 ou 450 picomoles pour 8x10⁸ globules rouges et/ou le niveau de 6-méthyl-mercaptopurine est supérieur à environ 5500, 5700, 6000, 6500, 7000, 7500 ou 8000 picomoles pour 8x10⁸ globules rouges.

5. Procédé selon la revendication 4, dans lequel ledit procédé est utile pour réduire et/ou surveiller la toxicité dudit traitement.

6. Procédé *in vitro* pour déterminer l'efficacité et la toxicité du traitement d'un sujet ayant un trouble gastro-intestinal à médiation immune ou une maladie auto-immune qui n'est pas une maladie inflammatoire de l'intestin (non-IBD) par administration d'un médicament à la 6-mercaptopurine, comprenant les étapes consistant à :
(a) déterminer *in vitro* un niveau de 6-thioguanine dans un échantillon dudit sujet ayant ledit trouble gastro-intestinal à médiation immune ou ladite maladie auto-immune qui n'est pas une maladie inflammatoire de l'intestin (non-IBD), et/ou
(b) déterminer *in vitro* un niveau de 6-méthyl-mercaptopurine dans un échantillon dudit sujet ayant ledit trouble gastro-intestinal à médiation immune ou ladite maladie auto-immune qui n'est pas une maladie inflammatoire de l'intestin (non-IBD),
dans lequel ledit traitement est considéré comme efficace si le niveau de 6-thioguanine est dans la plage d'environ 230 picomoles pour 8x10⁸ globules rouges à environ 400 picomoles pour 8x10⁸ globules rouges et dans lequel ledit traitement est considéré comme toxique si le niveau de 6-thioguanine est supérieur à environ 350, 370, 390, 400, 425 ou 450 picomoles pour 8x10⁸ et/ou le niveau de 6-méthyl-mercaptopurine est supérieur à environ 5500, 5700, 6000, 6500, 7000, 7500 ou 8000 picomoles pour 8x10⁸ globules rouges.

7. Procédé selon la revendication 6, dans lequel ledit niveau de 6-thioguanine est d'environ 240, 250, 260, 280 et 300 picomoles pour 8x10⁸ globules rouges.

8. Procédé selon la revendication 6 ou 7, dans lequel ledit procédé est utile pour optimiser et/ou surveiller l'efficacité et réduire et/ou surveiller la toxicité dudit traitement.

9. Procédé selon les revendications 4 à 8, dans lequel ledit niveau de 6-méthyl-mercaptopurine est supérieur à environ 7000 picomoles par 8x10⁸ globules rouges.

10. Procédé selon les revendications 1 à 9, dans lequel ledit trouble gastro-intestinal à médiation immune est choisi parmi le groupe constitué d'une colite lymphocytaire, d'une colite microscopique, d'une colite au collagène, d'une entéropathie auto-immune, d'une maladie gastro-intestinale allergique et d'une maladie gastro-intestinale éosinophile.

11. Procédé selon les revendications 1 à 10, dans lequel ledit niveau de 6-thioguanine est déterminé dans les globules rouges.

12. Procédé selon les revendications 1 à 11, dans lequel ledit niveau est déterminé en utilisant une chromatographie liquide haute pression.
